# EUROPEAN PATENT SPECIFICATION

(11) **EP 0 399 851 B1**
(45) Date of publication and mention of the grant of the patent: **20.11.1996**
(21) Application number: 90305764.4
(22) Date of filing: 25.05.1990
(51) Int. Cl.: C10L 3/10, C10G 21/00, C10G 25/00, C07C 13/615

(54) **Purification of hydrocarbonaceous fractions**
Reinigung von Kohlenwasserstofffraktionen
Purification de fraction hydrocarbonées

(30) Priority: 26.05.1989 US 358758; 26.05.1989 US 358760; 26.05.1989 US 358761; 26.05.1989 US 358759
(43) Date of publication of application: 28.11.1990
(73) Proprietor: MOBIL OIL CORPORATION, New York New York 10017 (US)
(72) Inventor: Alexander, Richard Alan, Mobile, Alabama 36693 (US); Knight, Charles Edward, Mobile, Alabama 36609 (US); Whitehurst, Darrell Duyane, Titusville, New Jersey 08560 (US)
(74) Representative: Colmer, Stephen Gary

(56) References cited:
- US-A- 2 666 500
- US-A- 3 094 569
- US-A- 3 567 791
- US-A- 3 649 702
- US-A- 4 083 772

## Description

This invention relates to the purification of hydrocarbonaceous fractions. It more particularly refers to separating diamondoid organic compounds from hydrocarbon streams containing such.

Many hydrocarbonaceous mineral streams contain some small proportion of diamondoid compounds. These high boiling, saturated, three-dimensional polycyclic organics are illustrated by adamantane, diamantane, triamantam and various side chain substituted homologues, particularly the methyl derivatives. These compounds have high melting points and high vapor pressures for their molecular weights and have recently been found to cause problems during production and refining of hydrocarbonaceous minerals, particularly natural gas, by condensing out and solidifying, thereby clogging pipes and other pieces of equipment. For a survey of the chemistry of diamondoid compounds, see Fort, Jr., Raymond C., The Chemistry of Diamond Molecules, Marcel Dekker, 1976.

In recent times, new sources of hydrocarbon minerals have been brought into production which, for some unknown reason, have substantially larger concentrations of diamondoid compounds. Whereas in the past, the amount of diamondoid compounds has been too small to cause operational problems such as production cooler plugging, now these compounds represent both a larger problem and a larger opportunity. The presence of diamondoid compounds in natural gas has been found to cause plugging in the process equipment requiring costly maintenance downtime to remove. On the other hand, these very compounds which can deleteriously affect the profitability of natural gas production are themselves valuable products.

According to the invention there are provided processes for extracting diamondoid compounds from gases and fluids in general as variously defined in independent claims 1, 11, 17, 23 and 28.

It has been found that distillate fuel oil fractions which have a significant aromatic compound content, such as monocyclic aromatics, are good solvents for diamondoid compounds and can be used as a wash for the equipment used in the production and refining of diamondoid containing fluids. Thus it is particularly preferred that the solvent is a distillate fuel oil, particularly an aromatics distillate fuel oil: kerosene, diesel fuel and heavy gasoline are particular examples of suitable oils, with diesel being the most preferred.

If the diamondoid compound-containing gas stream to be purified is rich in substituted higher boiling point adamantane homologues, the solvation and gas/liquid separation steps may produce a gas stream having acceptable purity. If, however, if the diamondoid compound-containing gas feedstream contains a substantial fraction of less substituted lower boiling point adamantane homologues, the silica gel sorption step may be necessary to achieve the desired gas product stream purity or extent of diamondoid recovery.

In the process defined in claim 17, the porous solid should have a defined set of properties viz: a pore system large enough and having a suitable shape to be receptive to the rather bulky diamondoid compounds. These diamondoid compounds are very bulky because they contain at least three (3) mutually fused cyclohexane rings.

It should be understood that the operation of this invention is not based exclusively on shape selective absorption phenomena, a well known and widely used attribute of most porous solids. Certainly shape selective absorption plays an important part in this process - a molecule which is too large to fit into the pore of a solid cannot be absorbed in that pore. However, it has been found that porous solids which conform to the properties hereinabove set forth absorb diamondoid compounds preferentially even with respect to smaller hydrocarbon compounds which would be believed to be more readily absorbed if considered on a pure size based shape selectivity alone.

Substantially hydrocarbonaceous fractions comprising diamondoid compounds are peculiarly suitable for separation by a thermal gradient diffusion process defined in claim 28. The applicability of this technique is dependent upon the fact that the diamondoid compounds exhibit a large change in viscosity relative to temperature, that is, their viscosity goes down significantly per degree of increase in temperature. In fact, their viscosity rate of change with temperature is substantially greater than that of other hydrocarbons of similar boiling point range.

Further features of the invention are defined in the dependent claims.

Reference is now made to the accompanying drawings, in which:
Figure 1 is a simplified schematic diagram showing the major processing steps in the process according to the invention.
Figure 2 is a schematic diagram showing the major processing steps in the process according to the invention;
Figure 3 is a schematic diagram showing the major processing steps in the process according to the invention;
Figure 4 is a block flow diagram of apparatus for aromatics-selective solvent extraction according to the invention;
Figures 5, 6 and 7 are gas chromatographic analyses;
Figure 8 is a schematic diagram showing the major processing steps in the process according to the invention;
Figure 9 is a schematic perspective view of an apparatus for use with the embodiment shown in figure 8; and
Figures 10 and 11 show the results of chromatographic analyses.

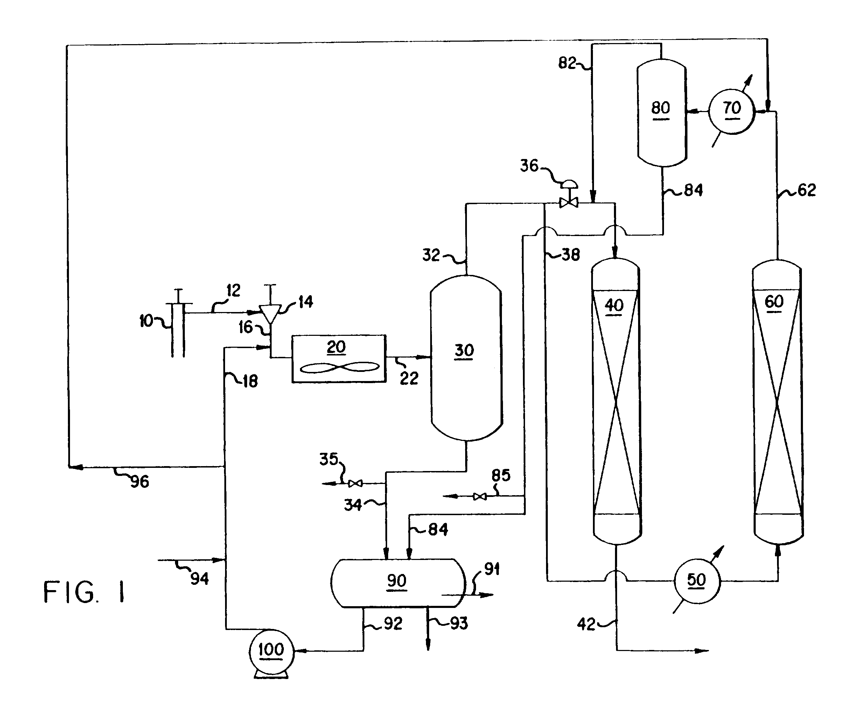

Refeming now to Figure 1, a first embodiment of the present invention is schematically illustrated. A diamondoid-laden natural gas stream 12 is withdrawn from wellhead 10 at high pressure, generally between about 3000 and 15000 psig (20.7 and 103 Mpa) typically around 11,000 psig (65.8 mPa). Pressure reduction valve 14, commonly referred to as a choke, reduces the natural gas pressure downstream of the choke to between about 900 and about 1400 psig (6.2 mPa and 9.7 mPa). Recycled solvent 18 is injected into the reduced pressure diamondoid-laden natural gas stream 16 upstream of process cooler 20 to prevent deposition of diamondoid solids within the cooler. Process cooler 20 is typically an air cooled exchanger with extended heat exchange tube surface area, commonly known as a fin-fan exchanger. In certain embodiments of the invention the solvent is a aromatic distillate.

Solvent injection rates of about 2 to 6 gallons per minute (GPM) (1.52 x 10⁻⁴ to 1.55 x 10⁻⁵ m³/s) at natural gas flowrates of 10 to 15 million standard cubic feet per day (MMSCF/D) (283000 to 425000 m³/day) have been found to be effective to reduce diamondoid deposition. Thus to achieve the desired diamondoid sorption in the added solvent, solvent charge rates of about 100 to 1000 gallons per million standard cubic feet of natural gas (G/MMSCF) (0.379 to 3.79 m³ per 28300 m³ of natural gas) are acceptable, and rates of between 200 and 800 G/MMSCF (0.757 to 3.03 m³ per 28300 m³ of natural gas) are preferred. The optimum charge rate within the disclosed ranges to minimize solvent costs while preventing diamondoid deposition in the downstream process equipment may be determined by one of ordinary skill in the art with a reasonable amount of trial and error.

If the solvent dosage selected for process operation is insufficient to maintain the diamondoids in solution through the process cooler, or if solvent injection is temporarily discontinued for operational reasons such as injection pump failure, diamondoids will likely be deposited on the inner surfaces of the process cooler heat exchange tubes, increasing the pressure drop across the air cooled exchanger. Thus one recommended method for determining optimum solvent dosage would be to monitor the change in natural gas pressure (ΔP) across the process cooler with respect to time. Any decrease in the ΔP across the process cooler would likely indicate diamondoid deposition on the inner surfaces of the cooler tubes and could be corrected with increased solvent dosage. The technique of monitoring heat exchanger operation by evaluating ΔP over time is well known to those skilled in the art of heat exchanger design and maintenance.

Depending on the concentration of diamondoid compounds in the natural gas stream as well as on the operating temperature and pressure, discontinuation of the solvent charge may precipitate partial or complete plugging of at least a portion of the process cooler heat exchange tubes. Such deposits may be removed via intermittent high dosage or "slug" solvent treatment. Slug solvent treatment has been found to be effective for removing diamondoid deposits from process cooler heat exchange tubes, e.g., charging 50 to 100 gallon slugs (0.19 to 0.379 m³) of solvent intermittently into the 10 to 15 MMSCF/D (283000 to 425000 m³/day) natural gas stream at a point upstream of the process cooler. The slugged solvent is then recovered by a method similar to that used for the continuously injected solvent, which method is described below.

The cooled mixture of natural gas and solvent 22 flows to production separator 30 where it is flashed to form an overhead vapor stream 32 and a bottom liquid stream 34. Production separator 30 is illustrated as a flash drum, i.e. a single stage vapor-liquid separation device, but may also comprise any suitable vapor-liquid separation apparatus known to those skilled in the art of process equipment design.

A first portion of the overhead vapor stream 32 flows through control valve 36 to enter sorption zone 40 while a second portion of the overhead vapor stream flow is preferably diverted by control valve 36 to form regeneration gas stream 38. The total overhead vapor stream may be charged to the sorption zone if an inert gas stream for use as a regeneration gas is both inexpensive and easily piped into the sorption process equipment. It is generally preferred, however, to use a portion of the overhead vapor stream as a regeneration gas to its inherent economy and availability. Regeneration gas flow to the silica gel sorption zone is preferably countercurrent, i.e. gas flow for silica gel desorption during regeneration should be oriented in the opposite direction from gas flow for silica gel sorption during the gas purification operation.

The first portion of the overhead vapor stream 32 then contacts a silica gel sorbent contained in sorption zone 40. The overhead vapor stream preferably flows downwardly in contact with the silica gel sorbent through the length of the sorption zone 40. Silica gel volume is preferably selected such that almost of the silica gel sorption capacity it utilized before regeneration.

The purified gas stream 42 is then withdrawn from sorption zone 40 and charged to pipeline or storage facilities. The second portion of the overhead vapor stream is preferably diverted for use as a regeneration gas as described above. Part of the purified gas stream 42 may be compressed and heated for use as a regeneration gas (compression equipment not shown). Generating silica gel using the purified gas effluent, for example from sorption zone 40, may prolong the silica gel useful life by decreasing the rate of steam deactivation. Regeneration gas 38 is heated in regeneration heat exchanger 50 to a temperature less than 315°C (600°F), preferably between about 177 and 288°C (350 and 550°F) and then charged to the bottom of sorption zone 60 to countercurrently desorb water and heavy hydrocarbons, particularly diamondoids, from the silica gel. The length of the regeration step is a function of regeneration gas temperature and flowrate as well as the amount of sorbed material contained in the silica gel sorption bed. These operating parameters may be varied to synchronize the regeneration cycle (desorption) of a first sorption zone with the gas purification cycle (sorption) of a second sorption zone. The sorption zones are preferably piped and valved in a parallel configuration such that one sorption zone may be operated in the gas purification mode while the other sorption zone is countercurrently regenerated.

Enriched regenerated gas 62 is cooled to a temperature of between about 24 and 60°C (75 and 140°F) in regeneration cooler 70 and its flashed in regeneration separator 80 to form an overhead gas stream 82 and a liquid bottom stream 84. The overhead gas stream is preferably recycled and mixed with the production separator overhead stream and purified in sorption zone 40. The regeneration separator overhead gas stream 82 may optionally be mixed with purified gas stream 42. While such optional configuration beneficially reduces the total gas flow through the sorption zone operating in the gas purification mode, it necessarily reduces both diamondoid compound recovery and natural gas product purity.

Liquid bottom stream 34 from production separator 30 and liquid bottom stream 84 from regeneration separator 80 normally flow to solvent accumulator drum 90. A portion of the diamondoid-containing solvent 91 is drawn off the solvent accumulator and fresh solvent 94 is added downstream to maintain diamondoid concentration in the solvent below saturation. In one embodiment the fresh solvent 94 may constitute purified solvent from stream 91. A water stream 93 is drawn off from solvent accumulator drum 90 and is sent to the process sewer for treatment and hydrocarbon recovery. The remaining diamondoid-containing solvent 92 is withdrawn from solvent accumulator drum 90, charged through pump 100 and mixed with the fresh solvent 94 to form recycled solvent stream 18 which is added to the natural gas stream 16 upstream from process cooler 20 as described above.

A slip stream of diamondoid-containing solvent 96 may optionally be diverted from recycled solvent stream 18 and mixed with the enriched regeneration gas stream 62 upstream of regeneration cooler 70. This slip stream addition to the enriched regeneration gas stream may be necessary to avoid diamondoid deposition in the regeneration gas cooler.

If the diamondoid contained in the feedstream to the present process consists predominately of adamantane and diamantane, it has been found that the two compounds may be effectively segregated and recovered separately. Given a feedstream in whcih the diamondoids principally consist of adamantane and diamantane, the liquid bottom streams from the production separator 30 and regeneration separator 80 have been found to be rich in diamantane and adamantane, respectively. Thus to recover the two compounds at relatively high purity, streams 35 and 85 are drawn off of streams 34 and 84, respectively, and are routed to separate diamondoid recovery processes (not shown).

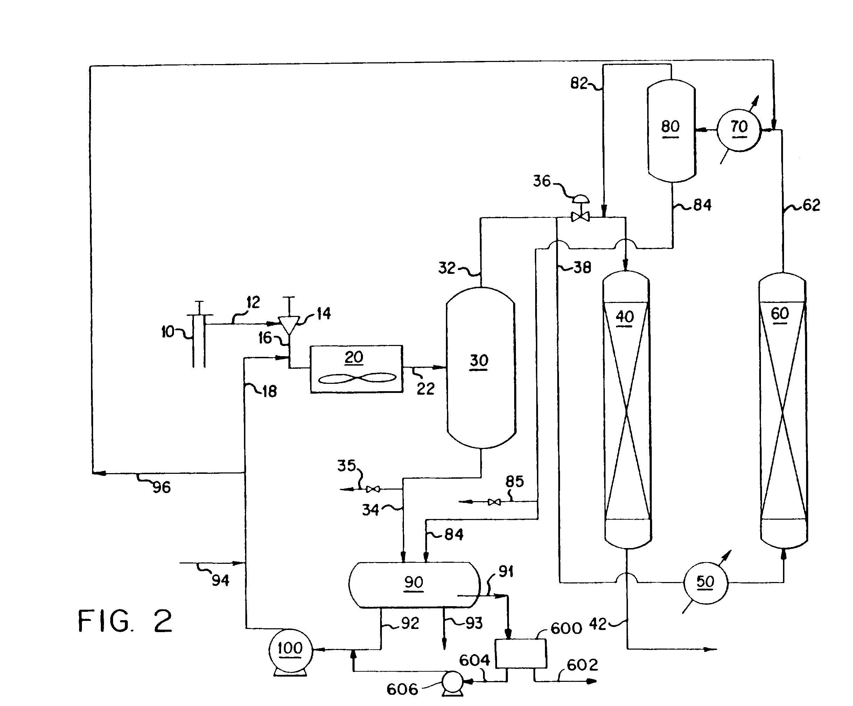

The process of figure 2 is similar to that of figure 1 and like parts are designated with like reference numerals.

The difference is that the diamondoid-containing stream 91 is contacted with a porous solid absorbent such as zeolite in a batch or continuous zeolite absorption process 600. The diamondoid compounds are then stripped off the zeolite absorbent and withdrawn in a diamondoid-enriched stream 602. The purified solvent stream 604 is then recycled through pump 606 into diamondoid containing solvent stream 92.

The porous solids having the proper, desirable pore structures and sizes adapted to be useful if this embodiment can be identified through theoretical considerations or by simple experimentation. Thus models, real or synthesized by a computer, can be constructed, as can models of diamondoid compounds. These models can be interacted to determine their compliance with the required critical parameters set forth above.

Alternatively, synthetic mixtures of diamondoid compounds (suitably equilibrium mixtures thereof) admixed with lighter (smaller) hydrocarbons, such as lower paraffins, can be contacted with various porous solids to determine practically which porous solids have the desired absorption properties. As noted, the best porous solid absorbents will absorb diamondoid compounds even preferentially to lighter aliphatics.

Another alternative approach to determining the applicability of any particular porous solid to use in this invention is a theoretical consideration of pore sizes and configurations of the porous solid compared to molecule sizes and configurations of the diamondoid compounds to be absorbed. The pore shapes and sizes of most porous solids have been thoroughly studied and published. Similarly, the shapes and dimensions of most known molecules have been measured and the results thereof published. Theoretical comparisons are therefore possible in many cases.

In many instances some combination of these described means of determining which porous solids to use in practising this invention will be found to be appropriate. Illustrative solids include zeolite crystals having pore structures composed of 24 to 36 atom rings. Of these ring atoms, half are chalcogens, e.g., oxygen and/or sulfur, and the other half are metal such as silicon, aluminum, boron, phosphorous, gallium, and/or iron. This list is illustrative and not limiting.

Zeolitic crystal structures containing some or all of these elements which have been found to be operative within the precepts of this embodiment include those which are commonly called 12 to 18 ring zeolites. Within this group, zeolithic structures referred to as faujasite, mazzite, offretite, mordenite, gmelinite, Linde L, ZSM-4, ZSM-12, ALPO-5, MAPSO-46, Co APO-50, VPI-5, zeolite beta and MCM-9 are illustrative of the types of crystal structures which are suited to use in this invention.

It is preferred to practise this invention with crystalline zeolite solids having interconnected, three dimensional channel/pore structures because this allows multiple access passageways into and out of the pore system thereby facilitating the absorption/desorption cycle upon which the practice of this invention relies. It is not, however limited to such three dimensional pore systems.

Suitable porous solids for use with the present invention typically have channel structures with minor radii or about 3-4 Angstroms. Porous solids having three dimensional pore systems useful with this embodiment typically include those solids having channel structures with minor radii of about 3-4 Angstroms and cage structures defined by the interconnecting channels with cage structure minor radii of about 6-8 Angstroms. For examples of these porous solids, see W.M. Meier and D.H. Olson, Atlas of Zeolite Structure Types, published by Butterworths on behalf of the Structure Commission of the International Zeolite Association, 1987.

The zeolite absorption aspects of the invention can be practised in a continuous process, in a batch process or in a hybrid, continuous-batch process. In a batch process, the diamondoid containing fluid, preferably liquid, is contacted with the absorbing porous solid for a time sufficient to reach absorption equilibrium, that is for the diamondoid compounds absorb out of the fluid into the porous solid. Upon reaching equilibrium, the solid and fluid are separated, and the porous solid treated to desorb the diamondoid compounds therefore. Upon all, or substantially all, of the diamondoid compounds being desorbed from the porous solid, it can be used to absorb additional diamondoid compounds, or it may need to be regenerated in order to make is reusable.

In a continuous process, diamondoid compound containing fluid may be continuously passed into contact with a fixed, fluidized or transport bed of suitable porous solid at a space velocity such that as much diamondoid compounds are desired is absorbed by the porous solid. In the case of a fixed bed absorber, the bed is periodically taken out of absorption service and regenerated to recover the diamondoid compound content thereof. A stirred bed reactor may be used in a similar way or it may have means to continuously or intermittently remove some of the porous solid from the bed for desorption while providing means to add make-up fresh or regenerated porous solid. A fixed-fluidized bed can operate similarly.

A transport bed reaction zone, by its fundamental nature, continuously removes porous solids from the absorption zone for desorption and recycling. In a fixed, stirred or fixed fluidized bed reaction zone type operation, multiple absorption zones can be used in a "swing bed" type operation where the feed is contacted with some bed or beds under absorption conditions while other bed or beds are being desorbed and/or regenerated.

The zeolite absorption zone according to this invention is suitably operated at a temperature of about 50 to 400°F (109 to 204°C), preferably at about 70 to 200°F (21 to 93°C). The pressure may be such as to keep the feed fluid and readily flowable. For example, pressures up to about 3000 psig (20.7 mPa) have been found to be operative. Contact times, expressed as space velocity, of about 1 to 30, preferably 2 to 10 LHSV have been found to be suitable. The combination of these operating parameters should be adjusted to produce whatever recovery and product purity is desired. Clearly longer contact times will absorb more diamondoid compounds but the purity of absorbate may be lower.

This invention is useful in lowering the concentration of diamondoid compound in the feed hydrocarbonaceous fluid as much s possible - in other words substantially removing all of the diamondoid compounds from the feed. To accomplish this with hydrocarbonaceous mineral fluid feeds may require a zeolite absorbent having as much as 10 times or more of absorption capacity than is actually absorbed in the zeolite before regeneration of the zeolite absorbent. In many cases a ratio of zeolite absorption capacity utilized to total zeolite absorption capacity of about 2 to 10, has been found suitable, while in other cases as low a ratio as 1.5 may be sufficient.

In situations where the diamondoid compound content of the porous solid is the limiting factor in the process, the ratio of absorption capacity utilized to the total absorption capacity can be as low as 0.5 or even lower, for example, 0.2 to 0.05. If it is desired to accomplish both results, that is remove much or substantially all the diamondoid compounds from the feed, and produce a product containing a very high diamondoid compound content, a multistep operation has been found to be effective. In this latter case, multiple beds of zeolite absorbent are sequenced so that the early bed(s) in the train are designed to remove substantially all the diamondoid compounds from the feed even at the expense of absorbate purity. When these beds are put into their desorption cycle, the desorbed effluent is passed through bed(s) designed to concentrate the diamondoid compounds, so that when these later beds are desorbed, a substantially purified and concentrated diamondoid compound product is produced.

Desorption of the absorbed diamondoid compounds can be accomplished by heating, steam stripping, washing with a selective solvent or combination thereof. Other known desorption techniques which suggest themselves may be used.

Where selective solvent washing is used to desorb the diamondoid absorbate from the porous solid representative solvents are illustrated by light paraffins, aromatic hydrocarbons, simple alcohols, lower ketones, ethers and carbon dioxide. This list is not exhaustive but merely illustrative. Preferred washing solvents include, in addition to the aforementioned carbon dioxide, propane, butanes, pentanes, hexanes, cyclohexanes, methyl cyclopentane, benzene, toluene, xylene, methanol, ethanol, propanols, butanols, acetone, methyl ethyl ketone, dimethyl ether, diethyl ether, methyl ethyl ether, mixtures of two or more of such compounds and/or fractions containing sufficiently high proportions of such compound(s) to be good washing solvents.

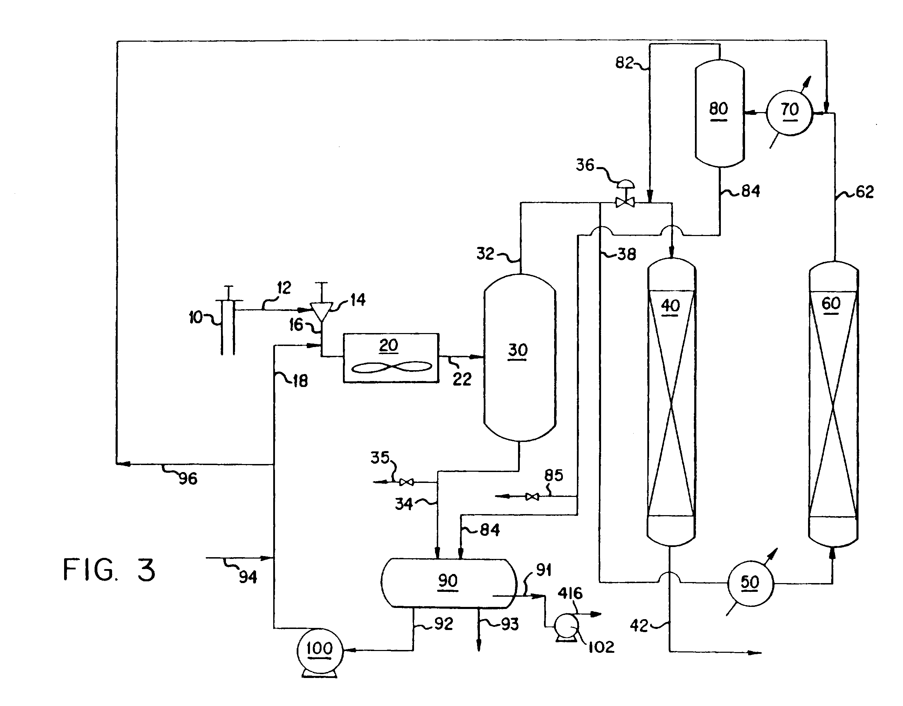

The embodiment of figure 3 is of particular importance when the solvent 18 is an aromatic distillate. The components of figure 3 are much the same as those of figure 1 and like parts have been designated with the reference numerals.

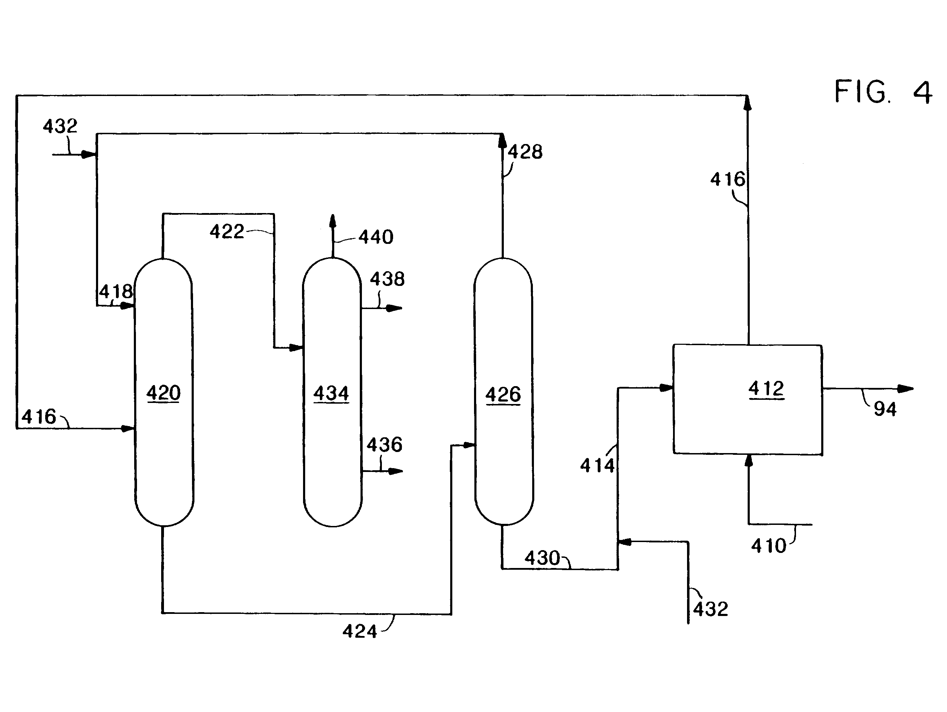

The only difference is that aromatic distillate 91 is fed to pump 102 from where it is pumped as aromatic extract 416. This is explained below with reference to figure 4.

The foregoing process description referring to figure 1 exemplifies one method by which a diamondoid containing aromatic distillate may be obtained for subsequent concentration by aromatics extraction as described below. The following process description details the steps by which a diamondoid-containing aromatic distillate stream may be treated to provide a stream rich in diamondoid compounds which may then be more earily purified to recover diamondoids.

Referring now to the Figure 4, a hydrocarbonaceous mineral source, or fraction thereof, such as natural gas, 410 is fed into a production/refining processing system 412. The hydrocarbonaceous mineral source is more preferably a diamondoid-laden natural gas stream as designated by numeral 12 in Figure 3, described above. The production/refining processing system described with reference to Figure 3 is a preferred embodiment of the production/refining processing system designated by numeral 412 in Figure 4.

As part of this processing, the equipment may periodically be treated with an aromatic distillate 414 as described above with reference to Figure 3 (equivalent to aromatic distillate stream 12 in figure 3). The hydrocarbon source 410 is preferably subjected to continuous washing with the aromatic distillate as described above. This washing/extraction process produces an extract 416 comprising diamondoid compounds, aromatic compounds not readily separable from the diamondoid compounds by distillation, linear aliphatic compounds and other compounds. The diamondoid compound containing stream 416 is then contacted with an aromatic-selective solvent feed 418 in an extraction zone 420 under extraction conditions so as to partition the aromatics away from the diamondoid compounds and produce a diamondoid rich, substantially aliphatic raffinate 422 and an aromatics rich, solvent extract 424.

The extract stream 424 is fed to a distillation column 426 which separates the stream into an aromatics-selective solvent distillate 428, and an aromatics-rich raffinate 430. The aromatics-rich raffinate 430 may have make-up aromatic distillate 432 added thereto if needed to reconstitute the aromatic distillate 414. The aromatics selective solvent 428 has make-up solvent 433 added thereto if needed to reconstitute the aromatic selective solvent feed 418.

The diamondoid containing raffinate 422 from the solvent extraction zone 420 is suitably fed to a distillation column 434 where it is resolved into a raffinate 436 rich in diamondoid compounds and other streams 438 and 440 rich in aliphatics of different boiling ranges. This invention is concerned with the production and recovery of the diamondoid compound fraction of the original hydrocarbonaceous feed. The recovery distillate streams 438 and 440 are merely illustrative of some other fractions which can be recovered in this distillation step. It is readily apparent that the number and content of the various distillate streams from this particular operation will be a function of the content of the feed and the particular product needs of the refiner.

The solvent extraction operation of this embodiment of the invention is itself as well known petroleum and petrochemical unit operation. Its application to the particularly described service constitutes an important part of this invention. Aromatic-selective solvents are well known and are illustrated by furfural, sulfolane, phenol, duosol, dimethyl formamide, etc. There are several well known commercially practised and available aromatics extraction processes, such as Udex, which may be quite suitable for use in this service.

One particularly important feature and attribute of this embodiment of the invention concerns corrosion inhibiting additives which are conventionally added to the distillate fuel oil wash liquid used to keep the producing and refining system clean of plugging deposits. It has quite surprisingly been found, much to the refiner's advantage, that the corrosion inhibitors frequently used in this service are recovered as a direct consequence of the aromatics extraction operation used herein. Thus it has been found that in extracting aromatics from admixture with diamondoids and other saturated organics, the corrosion inhibitors partition with the aromatics and are thereby separated and recovered from the diamondoid rich raffinate. Further, it has most advantageously been found that these corrosion inhibitors stay with the aromatics when the extract phase is later subjected to further resolution to separate the extracting solvent, solfolane for example, from the aromatics rich distillate fuel oil. Thus these expensive corrosion inhibitors continually recycle through the system along with the aromatics. Make-up may be required from time to time. However, the process saves and reuses large proportions of aromatic distillate-soluble corrosion inhibitors as opposed to the prior art practice of burning them along with the distillate fuel or solvent.

One example of an aromatic-distillate soluble corrosion inhibitor which is commonly used in this service is KP-151 brand corrosion inhibitor distributed by the Petrolite Company of St. Louis, MO.

The aromatics extraction step is suitably carried out with solvent used in excess, with respect to the stream being extracted, of about 2 to 1 to 5 to 1 ratio. Even higher excess solvent can be used. The particular solvent chosen for the extraction must be a good solvent for aromatic hydrocarbons and a poor solvent for saturated hydrocarbons. The solvent should preferably have a boiling point lower than the boiling point of the aromatics being extracted and preferably should not form an azeotrope with any of them.

The extraction temperature is suitably about 35 to 200°C (95 to 392°F), preferably about 60 to 100°C, (140 to 212°F). the temperature of the extraction process, and indeed the solvent to feed ratio, may be tailored to suit the particular type of operation preferred in any specific situation. It is usually preferred to run the extraction process at as high a temperature as possible, consistent with other operating limitations, in order to induce rapid equilibration of the feed and to prevent or at least retard crystallization of the high melting diamondoids, particulary as their concentration increases as the aromatics are extracted away. However, temperatures below the normal boiling point of the extraction solvent are preferred in order to avoid excessive pressures during the extraction process. Pressure is suitably controlled to maintin the aromatics extraction solvent and the aromatic distillate in the liquid phase at operating temperature, typically between atmospheric pressure and about 790 kPa (100 psig).

The temperature, pressure and ratio of solvent to feed all can be interdependently varied to adjust the proportion of diamondoids being recovered. Thus if it is desired to recover the most diamondoids possible from the feed, the temperature will be kept to a minimum consistent with all of the other parameters hereof, and the proportion of solvent will be kept toward the lower end of the range specified. To the contrary, when it is desired to operate this process to maximize throughput and merely to keep the production/refining equipment clean and unplugged, higher temperatures and higher extractant ratios will be used. In this way although lower proportions of diamondoids will be recovered, the process can be operated with higher throughputs and a good equilibrium of diamondoid concentration can be achieved sufficient to minimize plugging problems.

The extract phase from the aromatics extraction process is suitably resolved by fractional distillation in any conventional distillation column suitably designed to separate the extracting solvent from the aromatics/corrosion inhibitor extract. If the boiling points are far enough apart, flashing may be sufficient.

The raffinate, containing the diamondoid compound to be recovered, may also be resolved by distillation or steam stripping such that the diamondoids are recovered as raffinate. The type of distillation and the perfection of partition (recovery) is a matter of choice and design. Clearly the distillation operating parameters which will yield the largest diamondoid recovery will probably also yield the least pure diamondoid product. The contrary is also true, i.e., a purer diamondoid product stream will be achieved at the expense of the quantity recovered. If desired, a train of distillation means can be suitably used.

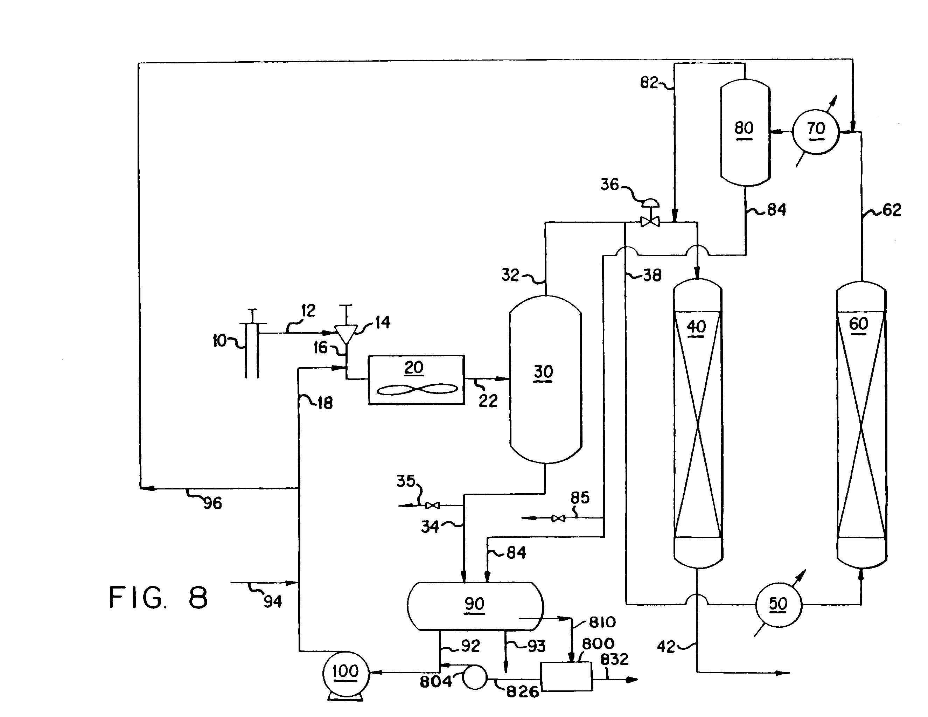

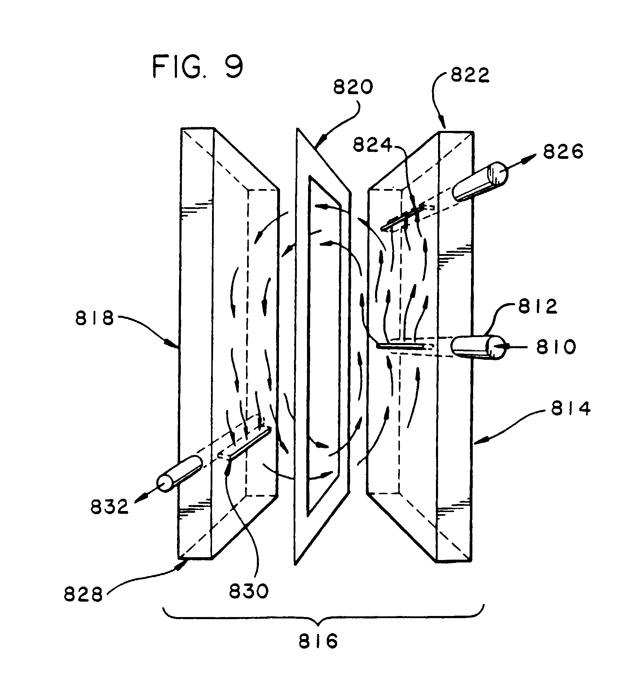

In figure 8, as in figure 1, liquid bottom stream 34 from production separator 30 and 84 from regeneration separator 80 normally flow to solvent accumulator drum 90. Many of the parts are similar and like parts are designated by like reference numerals. However, a portion of diamondoid-containing solvent 810 is drawn off the solvent accumulator 90 and charged to thermal diffusion unit 800. Partially purified solvent 826 is then charged through pump 804 and mixed with diamondoid-containing solvent 92 to be recycled. Thermal diffusion unit 800 is described below with reference to Figure 9.

Referring now to Figure 9, the aforementioned solution of diamondoid compounds 810 is fed to an intermediate height inlet 812 on the hotter side 814 of a thermal diffusion apparatus 816. The hot surface 814 is maintained at a temperature of up to about 500°F (260°C) and is spaced apart from a parallel cooler surface 818 maintained at a temperature of less than about 490°F (254°C). The space between the surfaces is set to between 0.003 and 0.01 inch (0.0076 and 0.0254 cm), which is maintained by a gasket 820 of that thickness interposed between the outer edges of the hot and cooler surfaces. Proximate to the top 822 of the hotter surface 814 is a channel 824 through which a stream of solvent 826, depleted in diamondoid compounds, is collected for recycle or other use. Proximate to the bottom 828 of the cooler surface 818 is a second channel 430 through which a concentrated stream of diamondoid compounds 832 is collected.

A thermal gradient diffusion process can operate in either a batch or continuous mode. The material to be resolved is placed between two (2) surfaces which differ in temperature. On factor affecting efficiency of resolution is the temperature of the two surfaces. Efficiency of separation is increased as a fraction of temperature, both the absolute temperature of both the surfaces, and the temperature of each surface relative to the other. It is preferred that the temperature of both surfaces be as high as practical, suitably about 50 to 500°F (10 to 260°C). It is most preferred that the temperatures of the surfaces be about 100 to 300°F (38 to 149°C).

With respect to the temperature differential between the two surfaces, this should be as great as practical, within the requirement that the temperature of both surfaces should be as high as practical. Suitably, the two surfaces should differ in temperature by at least about 10°F (5.6°C), preferably at least about 50°F (28°C). In accord with this embodiment of the invention then, the higher temperature surface is suitably maintained at a temperature of about 100 to 500°F (38 to 260°C), and the lower temperature surface is suitably maintained at a temperature of about 50 to 490°F (10 to 254°C), while maintaining the temperature differential as set forth above.

In accord with this embodiment of the invention, the two surfaces hereinbefore referred to, should preferably be spaced close together. It has been found that spacings of less than about 0.01 inch (0.0254 cm) are appropriate. For better separation results, preferred spacing between the surfaces are up to about 0.003 inch (0.0076 cm). The surfaces may, of course, vary in length, both in the direction of flow of the hydrocarbon feed containing the diamondoid compounds, and normal to the flow direction.

Differences in the density of molecules closer to the hotter surface and molecules closer to the cooler surface cause convection currents to be set up within the apparatus between the surfaces. The most efficient separations are accomplished where the distance between the two surfaces is less than the radius f curvature of these convection currents. If this parameter is maintained, the molecules will strike a proximate surface before they have an opportunity to circulate around past that surface toward the other one. It is desired that the diamondoid molecules strike the cooler surface, and run down it to a collection point, without eddying back around. It is possible to assist in destroying eddy convection currents by installing baffles or insulation between the surfaces such that these eddy currents are disrupted, but not sufficient to severely retard the flow of molecules between the surfaces.

Since this separation process is dependent upon temperature differences and density differences between molecules, the separations can be assisted by artificially increasing gravitational forces acting on the molecules. This can be accomplished by carrying out the process under artificial gravity, such as by the application of centrifugal force. Artificial gravity up to about 100 G forces seem to be suitable.

The process of this invention can be carried out in several different modes to accomplish different objectives. If it is desired to remove as much diamondoid compound as possible from a hydrocarbon fraction without too much concern for the purity of the diamondoid fraction of the product, residence time in the instant process should be made relatively short, e.g. about 10 minutes. Further, if this is the object, low ratios of process direction length of the surfaces to width of the surfaces are desired, e.g. about 2 to 1. Still further, the highest achievable temperature and the highest achievable temperature differential should be used.

On the other hand, if it is desired to produce a lower rate of diamondoid recovery, but produce a purer diamondoid product stream, higher length to width ratios can be used. In this aspect of this invention, length to width ratios of as much as about 100 to 1 have been found to be suitable, with temperature differentials of about 10 to 50°F (5.6 to 28°C) being acceptable, and higher temperature differentials being desirable.

It is important that the overall temperature of the operation be such that the adamantane compounds do not precipitate from the feed stream. Thus, the operating temperatures may be limited by the melting point of the highest melting diamondoid compound in the feed. Adamantane is the highest melting diamondoid, 296°C at atmospheric pressure, and so if the operating temperature is maintained above 296°C with the pressure at atmospheric or higher, this condition will be fulfilled. However, because of mutual solubilities of diamondoids, crystallization is often inhibited and temperatures lower than the melting point of the highest melting point diamondoid present can be used. It is of course possible to operate at higher or lower pressures than atmospheric, depending upon other desirata, particularly good process operation. Therefore the absolute temperature requirement will vary depending upon the pressure. In any case, the functional requirement that the diamondoid be maintained as a liquid will govern.

### Examples

In the following examples, parts and percentages are by volume unless expressly stated to be on some other basis.

### Embodiment 1

The following examples are representative of the process referred to above as embodiment 1 and shown in figure 1 of the accompanying drawings.

### Well No. 1

The production cooler (air cooled fin-fan heat exchanger) of Well No. 1 was taken out of survice due to unacceptably high pressure drop across the exchanger. The heat exchange tubes, particularly the first several passes, were found to be plugged with a crystalline deposit which was analyzed and determined to be a mixture of diamondoid compounds rich in adamantane and diamantane.

Day 1 - The tubes were flushed with diesel fuel until mechanically clean and returned to service.

Day 2 - Intermittent injection of 50-100 gallon (0.189 - 0.379m³) slugs of diesel fuel containing a minor amount of KW-151 and KP-111 brand corrosion inhibitors distributed by the Petrolite Company of St. Louis, MO was initiated upstream of the production cooler. The average slug injection rate ranged between 1 and 2 slugs per day. The natural gas flowrate from Well No. 1 remained between 10 and 15 MMSCF/D (283,000 to 425,000m³/day).

Day 173 - Slug injection was discontinued and continuous injection of diesel fuel at 2-4 GPM (1.51 x 10⁻⁴ to 3.03 x 10⁻³m³/s) was initiated.

Day 316 - No variation in process conditions indicative of accumulating diamondoid solids. Visual inspections of process internals revealed no further accumulation of diamondoid solids. Analysis of circulating diesel fuel shows progressive increase in diamondoid content further indicating successful ongoing diamondoid extration.

### Well No. 2

Day 2 - Intermittent injection of 50-100 gallon (0.189 - 0.379m³) slugs of diesel fuel containing a minor amount of KW-151 and KP-111 brand corrosion inhibitors was initiated upstream of the production cooler in Well No. 2. The average slug injection rate ranged between 1 and 2 slugs per day. The natural gas flowrate for Well No. 2 remained between 10 and 15 MMSCF/D (283,000 to 425,000m³/day).

Day 110 - Slug injection was discontinued and continuous injection of diesel fuel at 2-4 GPM (1.51 x 10⁻⁴ to 3.03 x 10⁻⁴m³/s) was initiated.

Day 168 - No variation in process conditions indicative of accumulating diamondoid solids. Visual inspects of process internals revealed no further accumulation of diamondoid solids. Analysis of circulating diesel fuel shows progressive increase in diamondoid content further indicating successful ongoing diamondoid extraction.

### Well No. 3

Day 2 - Intermittent injection of 50-100 gallon (0.189 - 0.379m³) slugs of diesel fuel containing a minor amount of KW-151 and KP-111 brand corrosion inhibitors as initiated upstream of the production cooler in Well No. 3. The average slug injection rate ranged between 1 and 2 slugs per day. The natural gas flowrate for Well No. 3 remained between 10 and 15 MMSCF/D (283,000 to 425,000m³/day).

Day 173 - Slug injection was discontinued and continuous injection of diesel fuel at 2-4 GPM (1.51 x 10⁴ to 3.03 x 10⁻⁴m³/s) was initiated.

Day 316 - No variation in process conditions indicative of accumulating diamondoid solids. Visual inspections of process internals revealed no further accumulation of diamondoid solids. Analysis of circulating diesel fuel shows progressive increase in diamondoid content further indicating successful ongoing diamondoid extraction.

### Embodiment 3

The following examples are representative of the process referred to above as embodiment 3 and shown in figures 3 to 7 of the accompanying drawings.

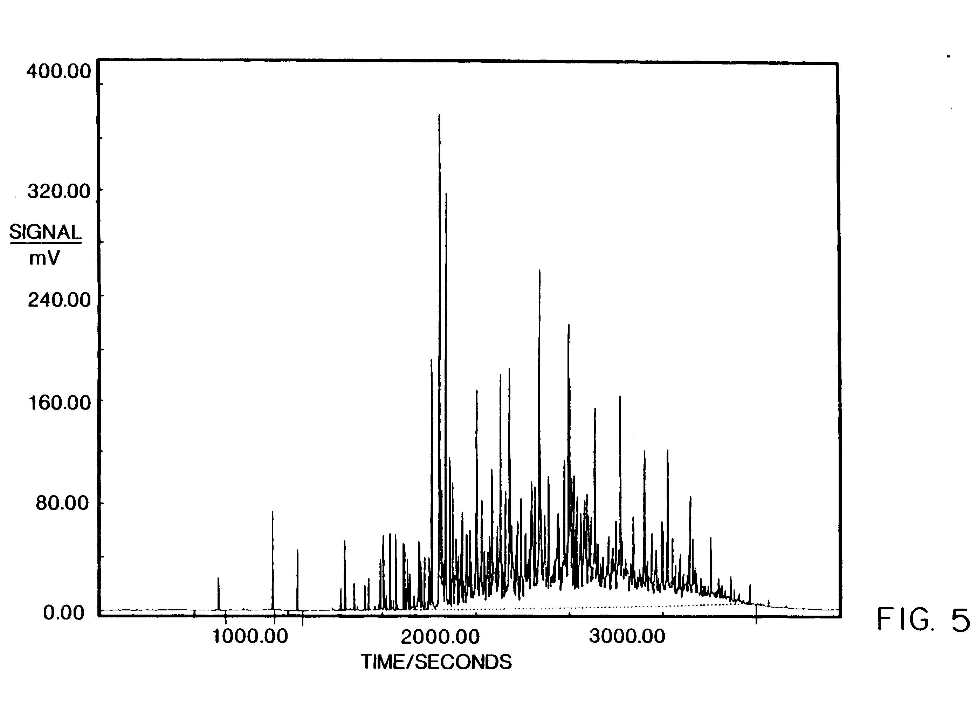

In the following examples a mixture of an equilibrium mixture of 10 parts of diamondoids dissolved in 90 parts of an aromatic distillate fuel oil containing 0.8 wt.% of KW-111 brand carboxylic acid/polyamine antifoam and 400 ppm wt. of KP-151 brand thioalkyl substituted phenolic heterocyclic corrosion inhibitor was used. The antifoam and corrosion inhibitor were purchased from the Petrolite Company of St. Louis, MO. The aromatic distillate fuel oil was a diesel fuel having an approximate composition as shown in the following Table. Fig. 5 shows a gas chromatographic analysis of the feed material.

| | |
|---|---|
| Aromatics | 46-58 wt. % |
| Paraffins | 22-29 wt. % |
| 1-ring Naphthenes | 12-18 wt. % |
| 2-ring Naphthenes | 5-6 wt. % |
| 3-ring Naphthenes | 1-3 wt. % |

### Example 1

Three hundred (300) parts of the feed mixture identified above was extracted with 600 parts of furfural at room temperature and atmospheric pressure in a batch extraction. After the mixture was shaken, and partitioned itself into layers, an additional 100 parts of furfural were added and the upper layer further extracted thereby. The saturated hydrocarbon (upper) layer was decanted off, washed with a mixture of 1 part methanol and 3 parts water to remove any lingering furfural, and then dried over alumina to yield 100 parts of diamondoid containing product.

The diamondoid containing product was resolved into its components in a gas chromatograph which analyzed the product to contain 15 parts of diamondoid. While the extract phase was not further resolved, it could have been conventionally separated to recover and recycle the furfural.

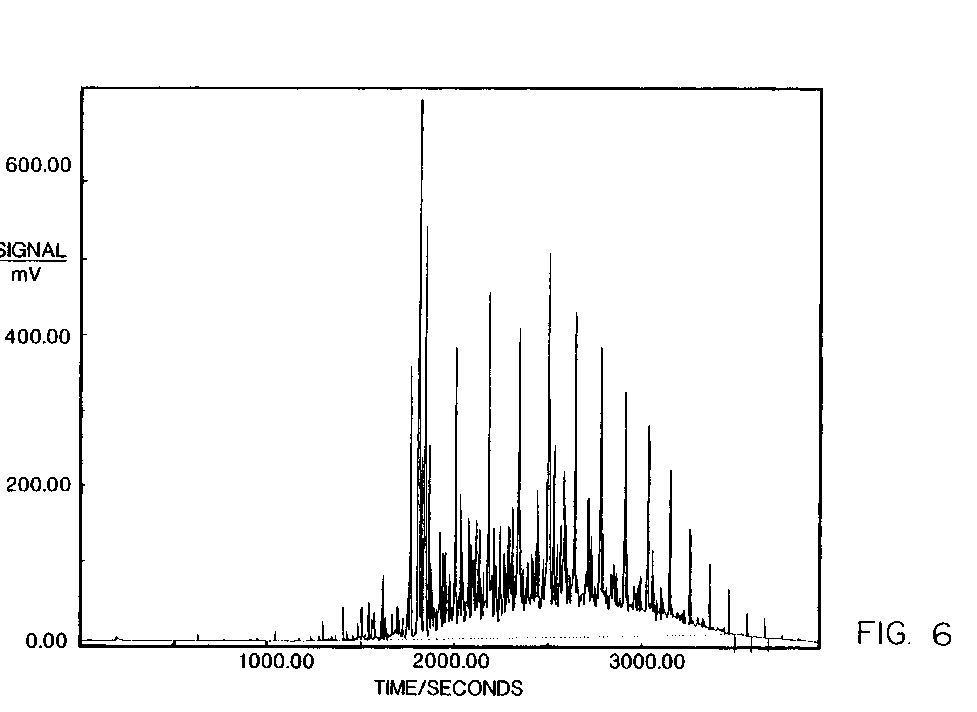

Fig. 6 shows a gas chromatographic analysis of the raffinate from the extraction unit.

### Example 2

This example was run on the same feed mixture as in Example I. About 30 parts of feed was extracted with 100 parts of dimethyl formamide at room temperature. The raffinate was subjected to gas chromatographic separation to show a yield of about 15 parts of diamondoids.

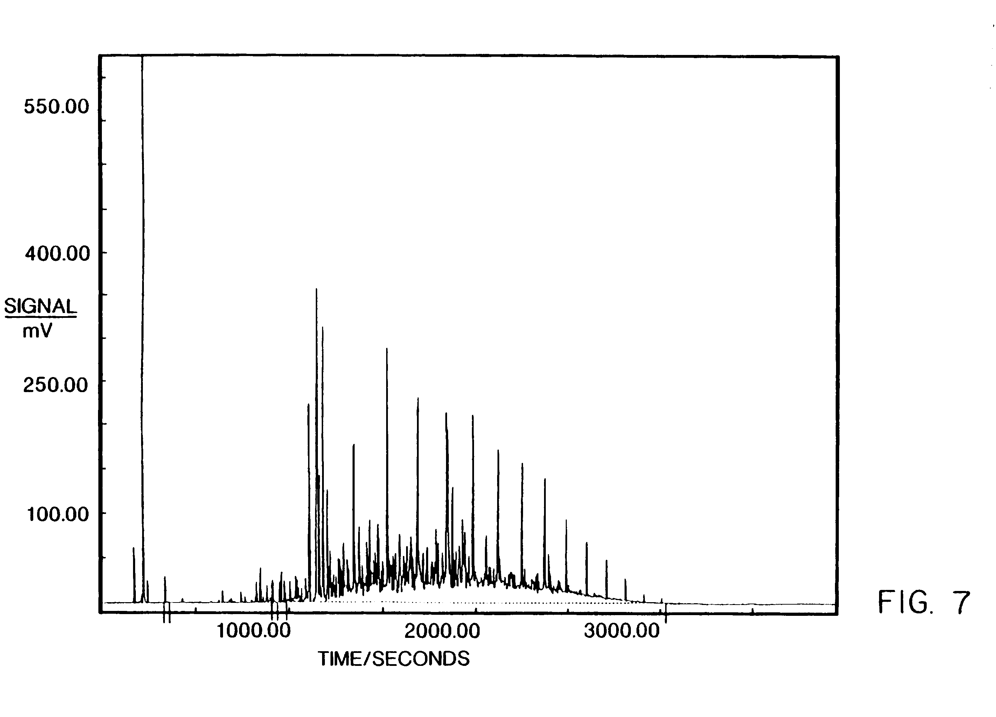

Figure 7 shows a gas chromatographic analysis of the raffinate from the extraction unit.

### Embodiment 4

The following examples are representative of the process referred to above as embodiment 4 and shown in figure 8 to 11 of the accompanying drawings.

In the following examples a mixture of an equilibrium mixture of 10 parts of diamondoids dissolved in 90 parts of an aromatic distillate fuel oil containing 0.8 wt. % of KW-111 brand carboxylic acid-polyamine antifoam and 400 ppm wt. of KP-151 brand thioalkyl substituted phenolic heterocyclic corrosion inhibitor was used. The antifoam and corrosion inhibitor were purchased from the Petrolite Company of St. Louis, MO. The aromatic distillate fuel oil was a diesel fuel having an approximate composition as shown in the following Table. Fig. 5 shows a gas chromatographic analysis of the feed material.

| | |
|---|---|
| Aromatics | 46-58 wt. % |
| Paraffins | 22-29 wt. % |
| 1-ring Naphthenes | 12-18 wt. % |
| 2-ring Naphthenes | 5-6 wt. % |
| 3-ring Naphthenes | 1-3 wt. % |

### Example 3

The diamondoid containing feed mixture of diamondoids in aromatic distillate fuel described above was resolved into its components in a gas chromatograph which analyzed the product to contain 10 parts of diamondoid in 90 parts of hydrocarbon liquid. The chromatographic analysis of the feed mixture is shown in Figure 5. The diamondoid containing product was then resolved in a thermal diffusion apparatus. The thermal diffusion apparatus included two concentric tubes approximately five (5) feet (1.52m) in length sized such that the outside diameter of the inner tube exceeded the inside diameter of the outer tube by approximately 0.006 inch (0.0076cm). Thus the space between the two surfaces was approximately 0.003 inch (0.0152cm). The inside diameter of the outer tube and the outside diameter of the inner tube were each approximately two (2) inches (5.08cm). The inner tube was maintained at a temperature of about 78°F (26°C) by flowing cooling water through the length of the inner tube. The outer tube was maintained at a temperature of about 148°F (64°C) by electric resistance heating.

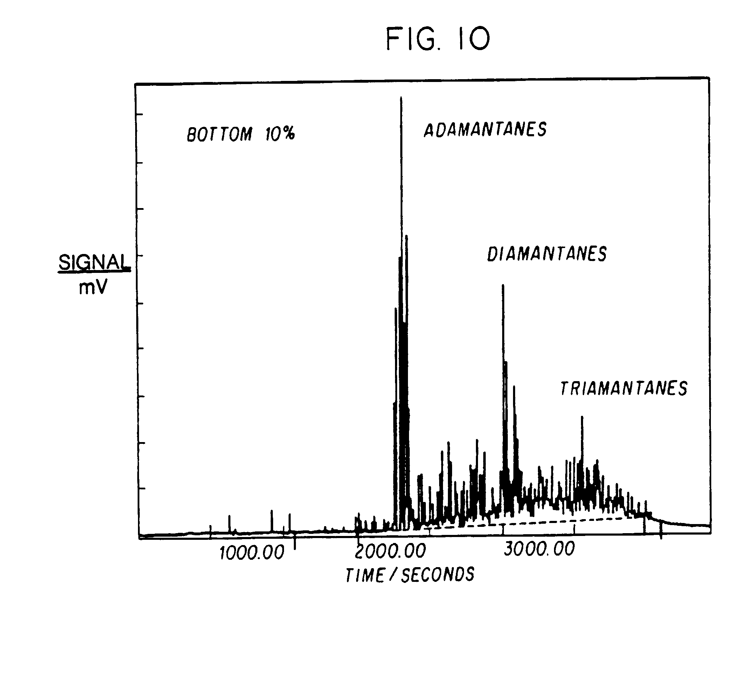

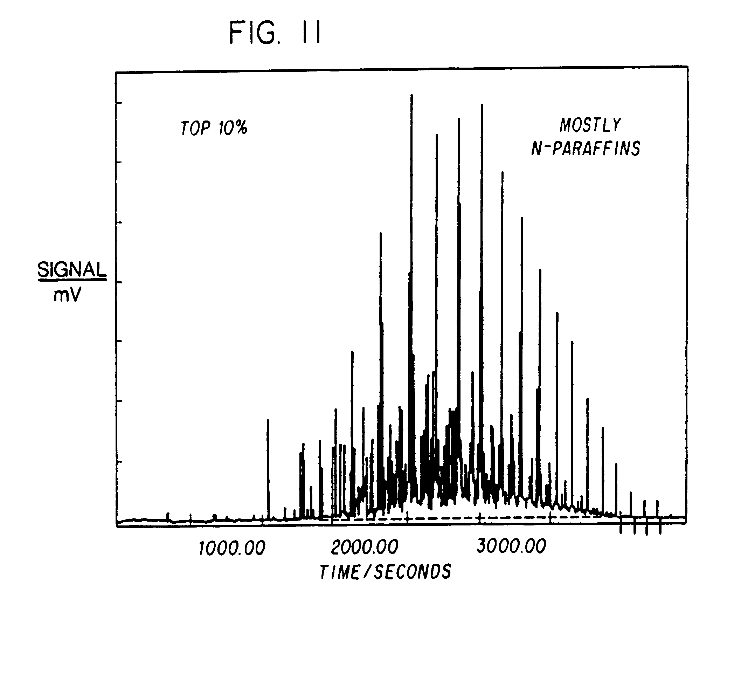

The diamondoid containing product mentioned above was then allowed to equilibrate in the thermal diffusion apparatus for a period of about 20 hours. The product was then sampled by withdrawing the top and bottom 10 volume percent from the thermal diffusion apparatus. The bottom 10% contained more than 20 weight percent diamondoid compounds as shown by chromatographic analysis in Figure 10. The top 10% predominately contained normal paraffins as shown by chromatographic analysis in Figure 11.

## Claims

1. A process for extracting diamondoid compounds from a gas stream comprising the of: -
(a) providing a gas stream containing a recoverable concentration of diamondoid compounds;
(b) subjeting the gas stream to a separation process whereby to separate at least part of said diamondoid compounds from the gas stream
wherein said separation process comprises the steps of:
(c) mixing said gas stream containing diamondoid compounds with a solvent in which diamondoid compounds are at least partially soluble;
(d) controlling the conditions including temperature and pressure of said mixture of step (c) to maintain at least a portion of said mixture in the liquid phase;
(e) separating said mixture under the controlled conditions of step (d) into a vapor stream and a diamondoid-enriched solvent stream; and
(f) recovering diamondoid compounds from said diamondoid-enriched solvent stream.

2. A process according to claim 1, wherein said solvent is a petroleum hydrocarbon, preferably diesel fuel.

3. A process according to claim 1 or 2, wherein step (e) further comprises cooling said mixture of step (d), preferably reducing the temperature of said mixture of step (d) to a temperature between about 24 and 60°C (75 and 140°F).

4. A process according to any preceding claim, wherein said separation process further includes the steps of:
(g) contacting said vapor stream with silica gel in a first sorption zone for a period of time sufficient for said silica gel to sorb at least a portion of said diamondoid compounds from said hydrocarbon gas; and
(h) recovering diamondoid compounds from said silica gel in a second sorption zone by contacting said silica gel with a regeneration fluid in which diamondoid compounds are at least partially soluble to desorb diamondoid compounds from said silica gel.

5. A process according to any of claims 1, 2, 3 or 4, wherein said diamondoid compounds include adamantane and diamantane; said solvent is selected to solvate diamantane preferentially to adamantane; in step (e) said mixture is separated into an adamantane-enriched gas stream and a diamantane-enriched solvent stream; in step (f) diamantane is recovered from said diamantane-enriched solvent stream and further comprising the steps of:
(g) contacting said adamantane-enriched gas stream with silica gel in a first sorption zone for a period of time sufficient for said silica gel to sorb at least a portion of adamantane from said adamantane-enriched gas; and
(h) recovering adamantane from silica gel in a second sorption zone by contacting said silica gel with a regeneration fluid in which adamantane is at least partially soluble to desorb adamantane from said silica gel.

6. A process according to claim 4, wherein after step (h), the following step is carried out:
recovering diamondoid compounds from at least a portion of said regeneration fluid by contacting said regeneration fluid with a porous solid having pore opening large enough to admit said diamondoid compounds thereinto and small enough so that at least about 50% of the external atoms of said diamondoid compounds are capable of simultaneously contacting the internal walls of the pores of said solid under conditions conducive to absorption of diamondoid compounds by said solid; and then desorbing the absorbate comprising diamondoid compounds from said solid absorbant.

7. A process according to any of claims 1 to 3, wherein after step (f) the following step is carried out:
recovering diamondoid compounds from said diamondoid-enriched solvent stream to produce a purified solvent stream by contacting said diamondoid-enriched solvent stream with a porous solid having pore opening large enough to admit said diamondoid compounds thereinto and small enough so that at least about 50% of the external atoms of said diamondoid compounds are capable of simultanously contacting the internal walls of the pores of said solid under conditions conducive to absorption of diamondoid compounds by said solid; and then desorbing the absorbate comprising diamondoid compounds from said porous solid.

8. A process according to any of claims 1 to 3, wherein the step of recovering diamondoid compounds from said diamondoid-enriched solvent stream comprises extracting said diamondoid-enriched solvent stream with an aromatics selective solvent to produce at least a diamondoid rich raffinate and an aromatics rich extract; and recovering diamondoid compounds from said diamondoid rich raffinate.

9. A process according to claim 8, wherein said partially purified gas stream is contacted with silica gel in a first sorption zone for a period of time sufficient for said silica gel to sorb at least a portion of said diamondoid compounds from said hydrocarbon gas;
said diamondoid compounds are recovered from silica gel in a second sorption zone by contacting said silica gel with a regeneration fluid in which diamondoid compounds are at least partially soluble to desorb diamondoid compounds from said silica gel;
said regeneration fluid is separated into a regeneration gas stream and a regeneration liquid stream; and
said diamondoid compounds are concentrated in said regeneration liquid stream by a method which comprises extracting said regeneration liquid stream with an aromatics selective solvent to produce at least a diamondoid rich raffinate and an aromatics rich extract; and recovering diamondoid compounds from said diamondoid rich raffinate.

10. A process according to any of claims 1 to 3, wherein the step of recovering diamondoid compounds from said diamondoid-enriched solvent stream comprises passing said diamondoid-enriched solvent stream between two surfaces spaced apart up to substantially 0.01 inch (0.0254 cm) at a temperature higher than the melting point of the lowest melting diamondoid in said diamondoid-enriched solvent stream up to substantially 500°F (260°C), and at a temperature differential between said surfaces of at least substantially 10°F (5.6°C) for a time sufficient to recover therefrom a first stream enriched in said diamondoid compounds, and a second stream depleted in said diamondoid compounds.

11. A process for extracting diamondoid compounds from a gas stream comprising the steps of:-
(a) providing a gas stream containing a recoverable concentration of diamondoid compounds;
(b) subjecting the gas stream to a separation process whereby to separate at least part of said diamondoid compounds from the gas stream;
wherein said separation process comprises the steps of:
(i) contacting said diamondoid-containing gas stream with silica gel in a sorption zone for a period of time sufficient for said silica gel to sorb at least a portion of said diamondoid compounds from said hydrocarbon gas; and
(j) regenerating said silica gel by contacting said silica gel with a regeneration fluid in which diamondoid compounds are at least partially soluble to desorb diamondoid compounds from said silica gel.

12. A process according to claim 11, wherein the gas stream is a natural gas stream.

13. A process according to claim 11 or 12, wherein after step (j) the following step is carried out:
recovering diamondoid compounds from at least a portion of said regeneration fluid by contacting said regeneration fluid with a porous solid having pore openings large enough to admit said diamondoid compounds thereinto and small enough so that at least about 50% of the external atoms of said diamondoid compounds are capable of simultaneously contacting the internal walls of the pores of said solid under conditions conducive to adsorption of diamondoid compounds by said solid; and then desorbing the absorbate comprising diamondoid compounds from said porous solid.

14. A process according to claim 11 or 12, wherein after step (j) the following steps are carried out:
separating said regeneration fluid into a regeneration gas stream and a regeneration liquid stream; and
concentrating diamondoid compounds in said regeneration liquid stream by a method which comprises extracting said regeneration liquid stream with an aromatics selective solvent to produce at least a diamondoid rich raffinate and an aromatics rich extract; and recovering diamondoid compounds from said diamondoid rich raffinate.

15. A process according to claim 11 or 12, wherein after step (j) the following step is carried out:
recovering diamondoid compounds from said diamondoid-enriched solvent stream by passing said diamondoid-enriched solvent stream between two surfaces spaced apart up to substantially 0.01 inch (0.0254 cm) at a temperature higher than the melting point of the lowest melting diamondoid in said diamondoid-enriched solvent stream up to substantially 500°F (260°C), and at a temperature differential between said surfaces of at least substantially 10°F (5.6°C) for a time sufficient to recover therefrom a first stream enriched in said diamondoid compounds, and a second stream depleted in said diamondoid compounds.

16. A process according to claim 4, 5, 6, 9, 11, 12, 13, 14 or 15, wherein said silica gel contacting step (g) or (i) is carried out under conditions of temperature and pressure to prevent substantial formation of solid diamondoid deposits in said sorption zone.

17. A process for extracting diamondoid compounds from a fluid comprising the steps of:-
(a) providing a fluid containing a recoverable concentration of diamondoid compounds;
(b) subjecting the fluid to a separation process whereby to separate at least part of said diamondoid compounds from the fluid;
wherein said separation process comprises the steps of:
contacting said fluid with a porous solid having pore openings large enough to admit said diamondoid compounds thereinto and small enough so that at least about 50% of the external atoms of said diamondoid compounds are capable of simultaneously contacting the internal walls of the pores of said solid to absorb diamondoid compounds by said solid; and then desorbing the absorbate comprising diamondoid compounds from said solid absorbant;
wherein said absorption is carried out at substantially 50 to 400°F (10 to 204°C) and/or said absorption is carried out at a pressure such that said fluid is a liquid.
and wherein:
(i) said porous solid is a zeolite solid comprising pores having from substantially 24 to 26 atoms defining at least one pore system; and/or.
(ii) said porous solid is a zeolite solid which comprises at least one of silicon, aluminum, boron, phosphorous, gallium or iron; and/or
(iii) said porous solid is a zeolite solid which has a topology corresponding to that of at least one of faujasite, mazzite, offretite, mordenite, gmelinite, Linde L, ZSM-12, ALPO-5, MAPSO-46, Co APO-50, VPI-5, zeolite beta, ZSM-4 or MCM-9; and/or
(iv) said porous solid contains channel structures having minor radii of substantially 3 to 4 Angstroms.

18. A process according to claim 17, wherein said absorption process is carried out at substantially 70 to 200 °F (21 to 93°C).

19. A process according to claim 17 or 18, comprising separating porous solid containing absorbate comprising diamondoid compounds from the fluid, and then preferably said separated porous solid is subsequently:
(i) heated for a time and at a temperature sufficient to desorb diamondoid compounds therefrom;
(ii) steam stripped to recover diamondoid compounds therefrom; or
(iii) washed with a solvent to leach said diamondoid compounds out of said porous liquid; and then said diamondoid compounds are separated from said solvent.

20. A process according to claim 19, wherein said solvent is at least one selected from the group consisting of propane, butanes, pentanes, hexanes, cyclohexane, methyl cyclopentane, benzene, toluene, xylene, methanol, ethanol, propanols, butanols, acetone, methyl ethyl ketone, dimethyl ether, diethyl ether, methyl ethyl ether and carbon dioxide and mixtures thereof.

21. A process according to claim 17 or 18, wherein at least a large fraction of diamondoid compounds are absorbed from said fluid as an impure absorbate in a first porous solid; said diamondoid compounds containing first porous solid is separated from said fluid; said absorbate is desorbed to form a first desorbate; diamondoid compounds are absorbed from said first desorbate in a second porous solid under conditions sufficient to produce an absorbate having a higher concentration of diamondoid compounds; said diamondoid compound containing second porous solid is separated from said first desorbate; and diamondoid compounds are desorbed from said second porous solid.

22. A process according to claim 21, wherein said first and second porous solids are the same, and/or said absorption is carried out in a fixed bed, a fixed fluidized bed, or in a transport bed, and/or there are at least two beds of solids, one operating in an absorption mode and the other operating in a desorption mode.

23. A process for extracting diamondoid compounds from a fluid comprising the steps of:-
(a) providing a fluid containing a recoverable concentration of diamondoid compounds;
(b) subjecting the fluid to a separation process whereby to separate at least part of said diamondoid compounds from the fluid;
wherein said fluid comprises a substantially hydrocarbon solution of said diamondoid compounds, said solution containing aromatic fractions; and wherein said separation process comprises concentrating said diamondoid compounds in said solution by extracting said solution with an aromatics selective solvent to produce at least a diamondoid rich raffinate and an aromatics rich extract, and recovering diamondoid compounds from said diamondoid rich raffinate.

24. A process according to claim 23, wherein said aromatics selective solvent is at least one selected from the group consisting of furfural, sulfolane, phenol, duosol and dimethyl formamide; and/or
said extraction is carried out at substantially 35 to 200°C (95 to 392°F) ; and/or
said extraction is carried out at an extractant to substantially hydrocarbon solution ratio of substantially 2 to 1 to 5 to 1.

25. A process according to claim 23 or 24, wherein said substantially hydrocarbon solution further contains at least one corrosion inhibitor.

26. A process according to claim 25, wherein said extraction partitions said corrosion inhibitor with said aromatics and away from said diamondoid compounds.

27. A process according to any of claims 23 to 26, wherein said hydrocarbon solution is formed by contacting a subsantially hydrocarbonaceous mineral containing diamondoid compounds with an aromatic rich distillate fuel oil, whereby to transport diamondoid compounds from said mineral to said oil.

28. A process for extracting diamondoid compounds from a fluid comprising the steps of:-
(a) providing a fluid containing a recoverable concentration of diamondoid compounds;
(b) subjecting the fluid to a separation process whereby to separate at least part of said diamondoid compounds from the fluid;
wherein said separation process comprises the steps of:
passing said fluid between two surfaces spaced apart up to substantially 0.01 inch (0.0254 cm) at a temperature higher than the melting point of the lowest melting diamondoid in said fluid up to substantially 500°F (260°C), and at a temperature differential between said surfaces of at least substantially 10°F (5.6°C) for a time sufficient to recover therefrom a first stream enriched in said diamondoid compounds, and a second stream depleted in said diamondoid compounds.

29. A process according to claim 28, wherein said surfaces are maintained at a temperature of substantially 50°F (10°C) to substantially 500°F (260°C); and/or
said surfaces are maintained at a temperature differential of substantially 100°F (38°C) to substantially 300°F (149°C).

30. A process according to claim 28 or 29, wherein natural gas containing a substantial concentration of diamondoid compounds is contacted with an aromatic distillate fuel oil for a time sufficient to extract diamondoid compounds from said natural gas and to form said fluid, which fluid comprises a solution of diamondoid compounds, and then passing said solution between said surfaces.

31. A process according to any of claims 28 to 30, wherein said fluid is sequentially passed between a multiplicity of said surface pairs in streamwise series, and a diamondoid-enriched product is recovered from each surface pair; or
said fluid is passed substantially simultaneously between a multiplicity of said surface pairs, and a diamondoid-enriched product is recovered from each surface pair.

32. A process according to any of claims 1 to 10, 17 to 22, or 28 to 31, wherein the fluid is a gas stream, preferably a natural gas stream.

33. A process according to any of claims 1 to 22 or 28 to 31, wherein the gas stream is obtained from a natural gas well, and said gas stream is provided by withdrawing natural gas containing diamondoid compounds from said natural gas well.

34. A process according to any of claims 17 to 22 or 28 to 31, wherein said fluid comprises natural gas liquids, or said fluid comprises a solution of said diamondoid compounds in distillate fuel oil.

## Patentansprüche

1. Verfahren zum Extrahieren diamantartiger Verbindungen aus einem Gasstrom, welches die Schritte umfaßt:
(a) Bereitstellen eines Gasstroms, der eine zur Rückgewinnung geeignete Konzentration an diamantartigen Verbindungen enthält;
(b) Unterziehen des Gasstromes einem Trennverfahren, wodurch zumindest ein Teil der diamantartigen Verbindungen vom Gasstrom abgetrennt wird,
wobei das Trennverfahren die Schritte umfaßt:
(c) Mischen des Gasstroms, der diamantartige Verbindungen enthält, mit einem Lösungsmittel, in dem die diamantartigen Verbindungen zumindest teilweise löslich sind;
(d) Regelung der Bedingungen, einschließlich Temperatur und Druck, der Mischung vom Schritt (c), wodurch zumindest ein Teil der Mischung in der flüssigen Phase gehalten wird;
(e) Trennen der Mischung bei den geregelten Bedingungen vom Schritt (d) in einen Dampfstrom und einen mit diamantartigen Verbindungen angereicherten Lösungsmittelstrom; und
(f) Gewinnen der diamantartigen Verbindungen aus dem mit diamantartigen Verbindungen angereicherten Lösungsmittelstrom.

2. Verfahren nach Anspruch 1, wobei das Lösungsmittel ein Benzinkohlenwasserstoff, vorzugsweise Dieselkraftstoff ist.

3. Verfahren nach Anspruch 1 oder 2, wobei der Schritt (e) außerdem das Abkühlen der Mischung vom Schritt (d) umfaßt, wobei die Temperatur der Mischung vom Schritt (d) vorzugsweise auf eine Temperatur zwischen etwa 24 und 60°C (75 und 140°F) verringert wird.

4. Verfahren nach einem der vorstehenden Ansprüche, wobei das Trennverfahren außerdem die Schritte umfaßt:
(g) Kontakt des Dampfstroms mit Kieselgel in einer ersten Sorptionszone während eines ausreichenden Zeitraums, damit das Kieselgel zumindest einen Teil der diamantartigen Verbindungen aus dem Kohlenwasserstoffgas sorbiert; und
(h) Gewinnen der diamantartigen Verbindungen aus dem Kieselgel in einer zweiten Sorptionszone durch Kontakt des Kieselgels mit einem Regenerierungsfluid, in dem die diamantartigen Verbindungen zumindest teilweise löslich sind, wodurch die diamantartigen Verbindungen aus dem Kieselgel desorbiert werden.

5. Verfahren nach einem der Ansprüche 1, 2, 3 oder 4, wobei die diamantartigen Verbindungen Adamantan und Diamantan umfassen; das Lösungsmittel so ausgewählt wird, daß Diamantan gegenüber Adamantan bevorzugt solvatisiert wird; die Mischung im Schritt (e) in einen mit Adamantan angereicherten Gasstrom und einen mit Diamantan angereicherten Lösungsmittelstrom getrennt wird; im Schritt (f) Diamanatan vom mit Diamantan angereicherten Lösungsmittelstrom gewonnen wird,
das außerdem die Schritte umfaßt:
(g) Kontakt des mit Adamantan angereicherten Gasstroms in einer ersten Zone während eines ausreichenden Zeitraums mit Kieselgel, damit Kieselgel zumindest einen Teil des Adamantans aus dem mit Adamantan angereicherten Gas sorbiert; und
(h) Gewinnen des Adamantans in einer zweiten Sorptionszone vom Kieselgel durch Kontakt von Kieselgel mit einem Regenerierungsfluid, in dem Adamantan zumindest teilweise löslich ist, wodurch Adamantan aus Kieselgel desorbiert wird.

6. Verfahren nach Anspruch 4, wobei nach dem Schritt (h) folgender Schritt vorgenommen wird:
Gewinnen der diamantartigen Verbindungen aus zumindest einem Teil des Regenerierungsfluids durch Kontakt des Regenerierungsfluids mit einem porösen Feststoff, dessen Porenöffnung ausreichend groß, damit die diamantartigen Verbindungen eindringen können, und ausreichend klein ist, damit zumindest etwa 50% der äußeren Atome der diamantartigen Verbindungen gleichzeitig die Innenwände der Poren des Feststoffs berühren können, bei Bedingungen, die die Absorption der diamantartigen Verbindungen durch den Feststoff einleiten; und anschließendes Desorbieren des Absorbats, das diamantartige Verbindungen umfaßt, aus dem festen Absorptionsmittel.

7. Verfahren nach einem der Ansprüche 1 bis 3, wobei nach dem Schritt (f) folgender Schritt vorgenommen wird: Gewinnen der diamantartigen Verbindungen aus dem mit diamantartigen Verbindungen angereicherten Lösungsmittelstrom, wodurch ein gereinigter Lösungsmittelstrom erzeugt wird, durch Kontakt des mit diamantartigen Verbindungen angereicherten Lösungsmittelstroms mit einem porösen Feststoff mit einer Porenöffnung, die ausreichend groß, damit die diamantartigen Verbindungen eindringen können, und ausreichend klein ist, damit zumindest etwa 50% der äußeren Atome der diamantartigen Verbindungen gleichzeitig die Innenwände der Poren des Feststoffs berühren können, bei Bedingungen, die die Absorption der diamantartigen Verbindungen vom Feststoff einleiten; und anschließendes Desorbieren des Absorbats, das diamantartige Verbindungen umfaßt, aus dem porösen Feststoff.

8. Verfahren nach einem der Ansprüche 1 bis 3, wobei der Schritt der Gewinnung der diamantartigen Verbindungen aus dem mit diamantartigen Verbindungen angereicherten Lösungsmittelstrom das Extrahieren des mit diamantartigen Verbindungen angereicherten Lösungsmittelstroms mit einem für Aromaten selektiven Lösungsmittel, wodurch zumindest ein an diamantartigen Verbindungen reiches Raffinat und ein aromatenreicher Extrakt erzeugt werden, und die Gewinnung der diamantartigen Verbindungen aus dem an diamantartigen Verbindungen reichen Raffinat umfaßt.

9. Verfahren nach Anspruch 8, wobei der teilweise gereinigte Gasstrom in einer ersten Sorptionszone ausreichend lange mit Kieselgel in Kontakt gebracht wird, damit das Kieselgel zumindest einen Teil der diamantartigen Verbindungen aus dem Kohlenwasserstoffgas sorbiert;
die diamantartigen Verbindungen in einer zweiten Sorptionszone vom Kieselgel gewonnen werden, indem das Kieselgel mit einem Regenerierungsfluid in Kontakt gebracht wird, in der die diamantartigen Verbindungen zumindest teilweise löslich sind, wodurch die diamantartigen Verbindungen aus dem Kieselgel desorbiert werden;
das Regenerierungsfluid in einen Regenerierungsgasstrom und einen Regenerierungsflüssigkeitsstrom getrennt wird; und
die diamantartigen Verbindungen durch ein Verfahren im Regenerierungsflüssigkeitsstrom nach einem Verfahren konzentriert werden, das das Extrahieren des Regenerierungsflüssigkeitsstroms mit einem für Aromaten selektiven Lösungsmittel, wodurch ein an diamantartigen Verbindungen reiches Raffinat und ein aromatenreicher Extrakt erzeugt werden, und die Gewinnung der diamantartigen Verbindungen aus dem an diamantartigen Verbindungen reichen Raffinat umfaßt.

10. Verfahren nach einem der Ansprüche 1 bis 3, wobei der Schritt zur Gewinnung der diamantartigen Verbindungen aus dem mit diamantartigen Verbindungen angereicherten Lösungsmittelstrom das Leiten des mit diamantartigen Verbindungen angereicherten Lösungsmittelstroms zwischen zwei im wesentlichen 0,01 inch (0,0254 cm) voneinander getrennten Oberflächen bei einer Temperatur oberhalb des Schmelzpunkts der diamantartigen Verbindung mit dem geringsten Schmelzpunkt im mit diamantartigen Verbindungen angereicherten Lösungsmittelstrom bis im wesentlichen 500°F (260°C) und bei einem Temperaturunterschied zwischen den Oberflächen von im wesentlichen mindestens 10°F (5,6°C) während eines ausreichenden Zeitraums umfaßt, wodurch ein erster mit diamantartigen Verbindungen angereicherter Strom und ein zweiter Strom gewonnen werden, der mit diamantartigen Verbindungen abgereichert ist.

11. Verfahren zum Extrahieren diamantartiger Verbindungen aus einem Gasstrom, welches die Schritte umfaßt:
(a) Bereitstellen eines Gasstroms, der eine für die Rückgewinnung geeignete Konzentration an diamantartigen Verbindungen enthält;
(b) Unterziehen des Gasstroms einem Trennverfahren, wodurch zumindest ein Teil der diamantartigen Verbindungen aus dem Gasstrom abgetrennt wird;
wobei das Trennverfahren die Schritte umfaßt:
(i) Kontakt des diamantartige Verbindungen enthaltenden Gasstroms in einer Sorptionszone ausreichend lange mit Kieselgel, damit das Kieselgel zumindest einen Teil der diamantartigen Verbindungen aus dem Kohlenwasserstoffgas sorbiert; und
(j) Regenerieren des Kieselgels durch Kontakt des Kieselgels mit einem Regenerierungsfluid, in dem die diamantartigen Verbindungen zumindest teilweise löslich sind, wodurch die diamantartigen Verbindungen aus dem Kieselgel desorbiert werden.

12. Verfahren nach Anspruch 11, wobei der Gasstrom ein Erdgasstrom ist.

13. Verfahren nach Anspruch 11 oder 12, wobei nach dem Schritt (j) folgender Schritt vorgenommen wird:
Gewinnen der diamantartigen Verbindungen von zumindest einem Teil des Regenerierungsfluids durch Kontakt des Regenerierungsfluids mit einem porösen Feststoff mit Porenöffnungen, die ausreichend groß, damit die diamantartigen Verbindungen eindringen können, und ausreichend klein sind, damit mindestens etwa 50% der äußeren Atome der diamantartigen Verbindungen gleichzeitig die Innenwände der Poren des Feststoffs berühren können, bei Bedingungen, die die Adsorption der diamantartigen Verbindungen durch den Feststoff einleiten; und anschließende Desorption des Absorbats, das diamantartige Verbindungen umfaßt, aus dem porösen Feststoff.

14. Verfahren nach Anspruch 11 oder 12, wobei nach dem Schritt (j) folgender Schritt vorgenommen wird:
Trennen des Regenerierungsfluids in einen Regenerierungsgasstrom und einen Regenerierungsflüssigkeitsstrom; und
Konzentrieren der diamantartigen Verbindungen im Regenerierungsflüssigkeitsstrom durch ein Verfahren, das das Extrahieren des Regenerierungsflüssigkeitsstroms mit einem für Aromaten selektiven Lösungsmittel, wodurch zumindest ein an diamantartigen Verbindungen reiches Raffinat und ein aromatenreicher Extrakt erzeugt werden, und das Gewinnen der diamantartigen Verbindungen aus dem an diamantartigen Verbindungen reichen Raffinat umfaßt.

15. Verfahren nach Anspruch 11 oder 12, wobei nach dem Schritt (j) folgender Schritt vorgenommen wird:
Gewinnen der diamantartigen Verbindungen aus dem mit diamantartigen Verbindungen angereicherten Lösungsmittelstrom durch Leiten des mit diamantartigen Verbindungen angereicherten Lösungsmittelstroms zwischen zwei bis zu 0,01 inch (0,0254 cm) voneinander getrennten Oberflächen bei einer Temperatur oberhalb des Schmelzpunkts der diamantartigen Verbindung mit dem geringsten Schmelzpunkt im mit diamantartigen Verbindungen angereicherten Lösungsmittelsstrom bis im wesentlichen 500°F (260°C) und bei einem Temperaturunterschied zwischen den Oberflächen von im wesentlichen mindestens 10°F (5,6°C) während eines ausreichenden Zeitraums, damit ein erster mit diamantartigen Verbindungen angereicherter Strom und ein zweiter Strom gewonnen werden, der mit diamantartigen Verbindungen abgereichert ist.

16. Verfahren nach Anspruch 4, 5, 6, 9, 11, 12, 13, 14 oder 15, wobei der Schritt des Kontaktes mit Kieselgel (g) oder (i) bei Temperatur- und Druckbedingungen vorgenommen wird, damit die wesentliche Entstehung fester diamantartiger Ablagerungen in der Sorptionszone vermieden wird.

17. Verfahren zum Extrahieren diamantartiger Verbindungen aus einem Fluid, welches die Schritte umfaßt:
(a) Bereitstellen eines Fluids, das eine für die Rückgewinnung geeignete Konzentration an diamantartigen Verbindungen enthält;
(b) Unterziehen des Fluids einem Trennverfahren, wodurch zumindest ein Teil der diamantartigen Verbindungen vom Fluid abgetrennt wird;
wobei das Trennverfahren die Schritte umfaßt:
Kontakt des Fluids mit einem porösen Feststoff mit Porenöffnungen, die ausreichend groß, damit die diamantartigen Verbindungen eindringen können, und ausreichend klein sind, damit mindestens etwa 50% der äußeren Atome der diamantartigen Verbindungen gleichzeitig die Innenwände der Poren des Feststoffs berühren können, wodurch die diamantartigen Verbindungen vom Feststoff absorbiert werden; und anschließendes Desorbieren des Absorbats, das die diamantartigen Verbindungen umfaßt, aus dem festen Absorptionsmittel;
wobei die Absorption bei im wesentlichen 50 bis 400°F (10 bis 204°C) erfolgt und/oder die Absorption bei einem Druck vorgenommen wird, so daß das Fluid eine Flüssigkeit ist,
und wobei:
(i) der poröse Feststoff ein Zeolithfeststoff ist, der Poren mit im wesentlichen 24 bis 26 Atomen aufweist, die mindestens ein Porensystem definieren; und/oder
(ii) der poröse Feststoff ein Zeolithfeststoff ist, der zumindest Silicium, Aluminium, Bor, Phosphor, Gallium oder Eisen enthält; und/oder
(iii) der poröse Feststoff ein Zeolithfeststoff mit einer Topologie ist, die zumindest einer der Topologien von Faujasit, Mazzit, Offretit, Mordenit, Gmelinit, Linde L, ZSM-12, ALPO-5, MAPSO-46, Co APO-50, VPI-5, Zeolith Beta, ZSM-4 oder MCM-9 entspricht; und/oder
(iv) der poröse Feststoff Kanalstrukturen mit Mindestradien von im wesentlichen 3 bis 4 Angström enthält.

18. Verfahren nach Anspruch 17, wobei das Absorptionsverfahren bei im wesentlichen 70 bis 200°F (21 bis 93°C) erfolgt.

19. Verfahren nach Anspruch 17 oder 18, das das Abtrennen des porösen, Absorbat enthaltenden Feststoffs, der diamantartige Verbindungen umfaßt, vom Fluid umfaßt, und der abgetrennte poröse Feststoff anschließend vorzugsweise:
(i) ausreichend lange und bei einer ausreichenden Temperatur erwärmt wird, damit die diamantartigen Verbindungen desorbiert werden;
(ii) mit Dampf gestrippt wird, damit die diamantartigen Verbindungen gewonnen werden; oder
(iii) mit einem Lösungsmittel gewaschen wird, damit die diamantartigen Verbindungen aus der porösen Flüssigkeit ausgewaschen werden, und die diamantartigen Verbindungen dann vom Lösungsmittel abgetrennt werden.

20. Verfahren nach Anspruch 19, wobei das Lösungsmittel mindestens eins aus der Gruppe von Propan, Butanen, Pentanen, Hexanen, Cyclohexan, Methylcyclopentan, Benzol, Toluol, Xylol, Methanol, Ethanol, Porpanolen, Butanolen, Aceton, Methylethylketon, Dimethylether, Diethylether, Methylethylether und Kohlendioxid und Mischungen davon ist.

21. Verfahren nach Anspruch 17 oder 18, wobei zumindest eine umfangreiche Fraktion der diamantartigen Verbindungen in einem ersten porösen Feststoff als unreines Absorbat aus dem Fluid absorbiert wird; der die diamantartige Verbindung enthaltende erste poröse Feststoff vom Fluid abgetrennt wird; das Absorbat desorbiert wird, wodurch ein erstes Desorbat entsteht; die diamantartigen Verbindungen in einem zweiten porösen Feststoff bei ausreichenden Bedingungen vom ersten Desorbat absorbiert werden, wodurch ein Absorbat mit einer stärkeren Konzentration diamantartiger Verbindungen erzeugt wird; der die diamantartige Verbindung enthaltende zweite poröse Feststoff vom ersten Desorbat abgetrennt wird; und die diamantartigen Verbindungen vom zweiten porösen Feststoff desorbiert werden.

22. Verfahren nach Anspruch 21, wobei der erste und der zweite poröse Feststoff gleich sind und/oder die Absorption in einem Festbett, einem stationären Wirbelbett oder in einem Förderbett erfolgt; und/oder mindestens zwei Betten der porösen Feststoffe vorliegen, von denen eins nach der Absorptionsart und das andere nach der Desorptionsart arbeiten.

23. Verfahren zum Extrahieren diamantartiger Verbindungen aus einem Fluid, welches die Schritte umfaßt:
(a) Bereitstellen eines Fluids, das eine für die Rückgewinnung geeignete Konzentration an diamantartigen Verbindungen enthält;
(b) Unterziehen des Fluids einem Trennverfahren, wodurch zumindest ein Teil der diamantartigen Verbindungen vom Fluid abgetrennt wird;
wobei das Fluid eine wesentliche Kohlenwasserstofflösung der diamantartigen Verbindungen umfaßt, die Lösung aromatische Fraktionen enthält, und das Trennverfahren das Konzentrieren der diamantartigen Verbindungen in der Lösung durch Extrahieren der Lösung mit einem für Aromaten selektiven Lösungsmittel, wodurch zumindest ein an diamantartigen Verbindungen Raffinat und ein aromatenreicher Extrakt erzeugt werden, und die Gewinnung der diamantartigen Verbindungen aus dem an diamantartigen Verbindungen reichen Raffinat umfaßt.

24. Verfahren nach Anspruch 23, wobei das für Aromaten selektive Lösungsmittel mindestens eins aus der Gruppe von Furfural, Sulfolan, Phenol, Duosol und Dimethylformamid ist; und/oder
die Extraktion bei im wesentlichen 35 bis 200°C (95 bis 392°F) erfolgt; und/oder
die Extraktion bei einem Verhältnis von Extraktionsmittel zur wesentlichen Kohlenwasserstofflösung von im wesentlichen 2:1 bis 5:1 erfolgt.

25. Verfahren nach Anspruch 23 oder 24, wobei die wesentliche Kohlenwasserstofflösung außerdem mindestens einen Korrosionsinhibitor enthält.

26. Verfahren nach Anspruch 25, wobei die Extraktion den Korrosionsinhibitor mit den Aromaten und von den diamantartigen Verbindungen abtrennt.

27. Verfahren nach einem der Ansprüche 23 bis 26, wobei die Kohlenwasserstofflösung durch Kontakt eines im wesentlichen kohlenwasserstoffhaltigen Minerals, das diamantartige Verbindungen enthält, mit einem aromatenreichen Destillatheizöl entsteht, wodurch die diamantartigen Verbindungen aus dem Mineral auf das Öl übertragen werden.

28. Verfahren zum Extrahieren diamantartiger Verbindungen aus einem Fluid, welches die Schritte umfaßt:
(a) Bereitstellen eines Fluids, das eine für die Rückgewinnung geeignete Konzentration an diamantartigen Verbindungen enthält;
(b) Unterziehen des Fluids einem Trennverfahren, wodurch zumindest ein Teil der diamantartigen Verbindungen aus dem Fluid abgetrennt wird;
wobei das Trennverfahren die Schritte umfaßt:
Leiten des Fluids zwischen zwei im wesentlichen 0,01 inch (0,0254 cm) voneinander getrennten Oberflächen bei einer Temperatur oberhalb des Schmelzpunkts der diamantartigen Verbindung im Fluid mit dem geringsten Schmelzpunkt bis im wesentlichen 500°F (260°C) und bei einem Temperaturunterschied zwischen den Oberflächen von im wesentlichen mindestens 10°F (5,6°C) während eines ausreichenden Zeitraums, damit ein erster mit diamantartigen Verbindungen angereicherter Strom und ein zweiter Strom gewonnen werden, der mit diamantartigen Verbindungen abgereichert ist.

29. Verfahren nach Anspruch 28, wobei die Oberflächen bei einer Temperatur von im wesentlichen 50°F (10°C) bis im wesentlichen 500°F (260°C) gehalten werden; und/oder
die Oberflächen bei einem Temperaturunterschied von im wesentlichen 100°F (38°C) bis im wesentlichen 300°F (149°C) gehalten werden.

30. Verfahren nach Anspruch 28 oder 29, wobei das Erdgas, das eine wesentliche Konzentration diamantartiger Verbindungen enthält, ausreichend lange mit einem aromatischen Destillatheizöl in Kontakt gebracht wird, damit die diamantartigen Verbindungen aus dem Erdgas extrahiert werden und das Fluid erzeugt wird, wobei das Fluid eine Lösung der diamantartigen Verbindungen umfaßt, und diese Lösung anschließend zwischen die Oberflächen geleitet wird.

31. Verfahren nach einem der Ansprüche 28 bis 30, wobei das Fluid in stromweiser Reihe nacheinander zwischen einer Anzahl dieser Oberflächenpaare geleitet und von jedem Oberflächenpaar ein mit diamantartigen Verbindungen angereichertes Produkt gewonnen wird; oder
das Fluid im wesentlichen gleichzeitig zwischen einer Anzahl dieser Oberflächenpaare geleitet und von jedem Oberflächenpaar ein mit diamantartigen Verbindungen angereichertes Produkt gewonnen wird.

32. Verfahren nach einem der Ansprüche 1 bis 10, 17 bis 22 oder 28 bis 31, wobei das Fluid ein Gasstrom, vorzugsweise ein Erdgasstrom ist.

33. Verfahren nach einem der Ansprüche 1 bis 22 oder 28 bis 31, wobei der Gasstrom von einem Erdgasbohrloch erhalten wird und der Gasstrom durch Abziehen von Erdgas, das diamantartige Verbindungen enthält, aus dem Erdgasbohrloch bereitgestellt wird.

34. Verfahren nach einem der Ansprüche 17 bis 22 oder 28 bis 31, wobei das Fluid Flüssigerdgas umfaßt oder das Fluid eine Lösung der diamantartigen Verbindungen in Destillatheizöl umfaßt.

## Revendications

1. Un procédé pour extraire des composés diamantoïdes, à partir d'un courant gazeux comprenant les étapes consistant à:
(a) amener un courant gazeux contenant une concentration récupérable de composés diamantoïdes;
(b) soumettre le mélange gazeux à un procédé de séparation de façon à séparer au moins une partie desdits composés diamantoïdes contenus dans le courant gazeux, ledit procédé de séparation étant caractérisé en ce qu'il comprend les étapes de:
(c) mélange dudit courant gazeux contenant les composés diamantoïdes avec un solvant dans lequel les composés diamantoïdes sont au moins partiellement solubles;
(d) contrôle des conditions, y compris la température et la pression dudit mélange de l'étape (c), afin de maintenir au moins une partie dudit mélange en phase liquide;
(e) séparation dudit mélange dans les conditions contrôlées de l'étape (d) en un courant vapeur et en un courant de solvant enrichi en composés diamantoïdes; et
(f) récupération des composés diamantoïdes contenus dans ledit courant de solvant enrichi en composés diamantoïdes.

2. Un procédé selon la revendication 1, dans lequel le dissolvant est un hydrocarbure de pétrole, de préférence un carburant diesel.

3. Un procédé selon la revendication 1 ou 2, dans lequel l'étape (e) comprend en outre le refroidissement dudit mélange de l'étape (d), de préférence en réduisant la température dudit mélange de l'étape (d) à une température comprise entre 24°C et 60°C (75 et 140°F).

4. Un procédé selon l'une quelconque des revendications précédentes, dans lequel ledit procédé de séparation inclut en outre les étapes de:
(g) contact dudit courant gazeux contenant lesdits composés diamantoïdes avec un gel de silice (silica gel) dans une première zone de sorption pendant une période de temps suffisante pour que ledit gel de silice sorbe au moins une partie desdits composés diamantoïdes contenus dans ledit courant gazeux hydrocarboné;
(h) récupération des composés diamantoïdes dudit gel de silice dans une deuxième zone de sorption par contact dudit gel de silice avec un fluide de régénération. dans lequel les composés diamantoïdes sont au moins partiellement solubles pour désorber les composés diamantoïdes dudit gel de silice.

5. Un procédé selon l'une quelconque des revendications 1, 2, 3 ou 4, dans lequel lesdits composés diamantoïdes incluent l'adamantane et le diamantane; dans lequel le solvant est choisi pour transformer par solvatation le diamantane de préférence en adamantane; dans lequel, dans l'étape (e), ledit mélange est séparé en un courant d'un gaz enrichi en adamantane et un courant de solvant enrichi en diamantane; dans lequel, dans l'étape (f), le diamantane est récupéré dudit courant enrichi en diamantane, ledit procédé comprenant en outre les étapes:
(g) de contact dudit courant gazeux enrichi en adamantane avec du gel de silice dans une première zone de sorption pendant une période de temps suffisante pour que ledit gel de silice puisse sorber au moins une partie de l'adamantane contenu dans ledit courant gazeux enrichi en adamantane; et
(h) de récupération de l'adamantane contenu dans le gel de silice dans une deuxième zone de sorption par contact dudit gel de silice avec un fluide de régénération, dans lequel l'adamantane est au moins partiellement soluble pour désorber l'adamantane dudit gel de silice.

6. Un procédé selon la revendication 4, dans lequel, après l'étape (h), est mise en oeuvre l'étape suivante:
récupération des composés diamantoïdes d'au moins une portion dudit fluide de régénération par contact du fluide de régénération avec un solide poreux présentant des ouvertures de pores suffisamment larges pour y admettre lesdits composés diamantoïdes et suffisamment petites pour qu'au moins environ 50% des atomes extérieurs desdits composés diamantoïdes soient susceptible d'entrer simultanément en contact avec les parois internes des pores dudit solide dans des conditions conduisant à l'absorption desdits composés diamantoïdes par ledit solide, puis ensuite à désorber l'adsorbant comprenant les composés diamantoïdes dudit absorbant solide.

7. Un procédé selon l'une quelconque des revendications 1 à 3, dans lequel, après l'étape (f) est mis en oeuvre l'étape suivante:
récupération des composés diamantoïdes dudit courant de solvant enrichi en composés diamantoïdes pour produire un courant de solvant purifié par contact dudit courant de solvant enrichi en composés diamantoïdes avec un solide poreux présentant des ouvertures de pores suffisamment larges pour y admettre des composés diamantoïdes et suffisamment petites pour qu'au moins environ 50% des atomes extérieurs desdits composés diamantoïdes soient susceptibles d'entrer simultanément en contact avec les parois internes des pores dudit solide dans des conditions conduisant à l'absorption des composés diamantoïdes par ledit solide, puis à désorber l'adsorbant comprenant les composés diamantoïdes dudit solide poreux.

8. Un procédé selon l'une quelconque des revendications 1 à 3, dans lequel l'étape de récupération des composés diamantoïdes contenus dans ledit courant de solvant enrichi en composés diamantoïdes comprend l'extraction dudit courant de solvant enrichi en composés diamantoïdes avec un solvant sélectif vis-à-vis des composés aromatiques pour produire au moins un raffinat enrichi en composés diamantoïdes et un extrait enrichi en composés aromatiques et récupérer les composés diamantoïdes dudit raffinat enrichi en composés diamantoïdes.

9. Un procédé selon la revendication 8, dans lequel le courant de gaz partiellement purifié est mis en contact avec du gel de silice dans une première zone de sorption pendant une période de temps suffisante pour que le gel de silice absorbe au moins une partie desdits composés diamantoïdes contenus dans ledit courant de gaz hydrocarboné;
lesdits composés diamantoïdes sont récupérés du gel de silice dans une deuxième zone de sorption par contact dudit gel de silice avec un fluide de régénération, dans lequel les composés diamantoïdes sont au moins partiellement solubles pour désorber les composés diamantoïdes dudit gel de silice;
ledit fluide de régénération est séparé en un courant de gaz de régénération et un courant de liquide de régénération, et
lesdits composés diamantoïdes sont concentrés dans le courant du liquide de régénération par une méthode qui consiste à extraire le courant liquide de régénération par un solvant sélectif vis-à-vis des composés aromatiques pour produire au moins un raffinat enrichi en composés diamantoïdes et un extrait enrichi en composés aromatiques, et à récupérer lesdits composés diamantoïdes dudit raffinat enrichi en composés diamantoïdes.

10. Un procédé selon l'une quelconque des revendications 1 à 3, dans lequel l'étape de récupération des composés diamantoïdes contenus dans ledit courant de solvant enrichi en composés diamantoïdes comprend le passage dudit courant enrichi en composés diamantoïdes entre deux surfaces espacées d'environ 0,0254 cm (0,01 inch) à une température supérieure à à celle du composé diamantoïde à point de fusion le plus bas contenu dans ledit courant de solvant enrichi en composés diamantoïdes jusqu'à pratiquement 260°C (500°F), et à un différentiel de température entre lesdites surfaces d'au moins environ 5,6°C (10°F) pendant une durée suffisante pour en récupérer un premier courant enrichi en composés diamantoïdes et un deuxième courant appauvri en composés diamantoïdes.

11. Un procédé d'extraction de composés diamantoïdes d'un courant gazeux comprenant les étapes consistant à:
(a) amener un courant gazeux contenant une concentration récupérable en composés diamantoïdes;
(b) soumettre le courant gazeux à un procédé de séparation dans lequel on sépare au moins une partie desdits composés diamantoïdes contenus dans ledit courant gazeux;
ledit procédé de séparation étant caractérisé en ce qu'il comprend les étapes de:
(i) contact dudit courant gazeux contenant lesdits composés diamantoïdes avec un gel de silice (silica gel) dans une première zone de sorption pendant une période de temps suffisante pour que ledit gel de silice sorbe au moins une partie desdits composés diamantoïdes contenus dans ledit courant gazeux hydrocarboné;
(j) régénération dudit gel de silice par contact de ce gel de silice avec un fluide de régénération dans lequel les composés diamantoïdes sont au moins partiellement solubles pour désorber les composés diamantoïdes dudit gel de silice.

12. Un procédé selon la revendication 11, dans lequel le courant gazeux est un courant de gaz naturel.

13. Un procédé selon la revendication 11 ou 12, dans lequel, après l'étape (j) est mise en oeuvre l'étape suivante:
récupération des composés diamantoïdes d'au moins une partie du fluide de régénération par contact dudit fluide de régénération avec un solide poreux présentant des ouvertures de pores suffisamment larges pour y admettre des composés diamantoïdes et suffisamment petites pour qu'au moins environ 50% des atomes extérieurs desdits composés diamantoïdes soient susceptibles d'entrer simultanément en contact avec les parois internes des pores dudit solide dans des conditions conduisant à l'absorption des composés diamantoïdes par ledit solide, puis à désorber l'adsorbant comprenant les composés diamantoïdes dudit solide poreux.

14. Un procédé selon la revendication 11 ou 12 dans lequel, après l'étape (j) sont mises en oeuvre les étapes suivantes:
séparation dudit fluide de régénération en un courant de gaz de régénération et un courant de liquide de régénération,
concentration desdits composés diamantoïdes dans le courant du liquide de régénération par une méthode qui consiste à extraire le courant liquide de régénération par un solvant sélectif vis-à-vis des composés aromatiques pour produire au moins un raffinat enrichi en composés diamantoïdes et un extrait enrichi en composés aromatiques, et à récupérer lesdits composés diamantoïdes dudit raffinat enrichi en composés diamantoïdes.

15. Un procédé selon l'une des revendications 11 ou 12, dans lequel, après l'étape (j), est mise en oeuvre l'étape suivante:
récupération des composés diamantoïdes contenus dans ledit courant de solvant enrichi en composés diamantoïdes comprend le passage dudit courant enrichi en composés diamantoïdes entre deux surfaces espacées d'environ 0,0254 cm (0,01 inch) à une température supérieure à à celle du composé diamantoïde à point de fusion le plus bas contenu dans ledit courant de solvant enrichi en composés diamantoïdes jusqu'à pratiquement 260°C (500°F), et à un différentiel de température entre lesdites surfaces d'au moins environ 5,6°C (10°F) pendant une durée suffisante pour en récupérer un premier courant enrichi en composés diamantoïdes et un deuxième courant appauvri en composés diamantoïdes.

16. Un procédé selon l'une quelconque des revendications 4, 5, 6, 9, 11, 12, 13, 14 ou 15, dans lequel ladite étape de contact du gel de silice (g) ou (i) est mise en oeuvre dans des conditions de température et de pression permettant d'empêcher une formation substantielle de dépôts diamantoïdes solides dans ladite zone de sorption.

17. Un procédé d'extraction de composés diamantoïdes d'un fluide comprenant les étapes consistant à:
(a) amener-un fluide contenant une concentration récupérable de composés diamantoïdes;
(b) à soumettre le mélange gazeux à un procédé de séparation de façon à séparer au moins une partie desdits composés diamantoïdes du courant gazeux,
dans lequel ledit procédé de séparation comprend les étapes de:
contact dudit fluide avec un solide poreux présentant 1 des ouvertures de pores suffisamment larges pour y admettre lesdits composés diamantoïdes et suffisamment petites pour qu'au moins environ 50% des atomes extérieurs desdits composés diamantoïdes soient susceptibles d'entrer simultanément en contact avec les parois internes des pores dudit solide pour réaliser l'absorption desdits composés diamantoïdes par ledit solide, puis ensuite à désorber l'adsorbant comprenant les composés diamantoïdes dudit absorbant solide;
dans laquelle ladite absorption est mise en oeuvre à une température comprise essentiellement entre 10 et 204°C (50 à 400°F) et/ou ladite absorption est mise en oeuvre à une pression telle que le fluide soit à l'état liquide, et dans laquelle:
(i) ledit solide poreux est une zéolite solide comprenant des pores formés d'environ 24 à 26 atomes définissant au moins un système formé de pores; et/ou
(ii) ledit solide poreux est une zéolite solide comprenant au moins un atome de silicium, d'aluminium, de bore, de phosphore, de gallium ou de fer; et/ou
(iii) ledit solide poreux est une zéolite solide qui présente une topologie correspondant à celle d'au moins une produits du groupe comprenant faujasite, mazzite, offretite, mordenite, gmelinite, Linde L, ZSM-12, ALPO-5, MAPSO-46, Co APO-50, VPI-5, zéolite beta, ZSM-4 ou MCM-9; et/ou
(iv) ledit solide poreux contient des structure en forme de canaux présentant des rayons faibles d'environ 3 à 4 Angstroems.

18. Procédé selon la revendication 17, dans laquelle ledit procédé d'absorption est effectué à une température de l'ordre de 21 à 93°C (70 à 200°F).

19. Procédé selon la revendication 17 ou 18, comprenant la séparation de l'adsorbant contenant le solide poreux comprenant des composés diamantoïdes du fluide, puis ensuite à soumettre ledit solide poreux ainsi séparé de préférence:
(i) à un chauffage pendant une durée et à une température suffisantes pour désorber les composés diamantoïdes qu'il contient;
(ii) à un entraînement à la vapeur pour récupérer les composés diamantoïdes qu'il contient, ou
(iii) à n lavage avec un solvant, pour entraîner par lavage lesdits composés diamantoïdes dudit liquide poreux, puis à séparer les composés diamantoïdes séparés dudit solvant.

20. Un procédé selon la revendication 19, dans lequel ledit solvant est au moins un des composés choisis dans le groupe comprenant propane, butanes, pentanes, hexanes, cyclohexane, méthyl cyclopentane, benzène, toluène, xylène, méthanol, éthanol, propanols, butanols, acétone, méthyl éthyl cétone, diméthyl éther, diéthyl éther, méthyl éthyl éther et dioxyde de carbone ainsi que leurs mélanges.

21. Procédé selon la revendication 17 ou 18, dans lequel au moins une large fraction des composés diamantoïdes sont absorbés dudit fluide sous forme d'un adsorbant impur sur un premier solide poreux; ledit composé diamantoïde contenant le premier solide poreux est séparé dudit fluide; ledit adsorbant est désorbé pour former un premier désorbat; les composés diamantoïdes sont absorbés dudit premier désorbat sur un second solide poreux dans des conditions suffisantes pour produire un adsorbant présentant une concentration supérieure en composés diamantoïdes; le composé diamantoïde contenant le second solide poreux est séparé dudit premier désorbat et les composés diamantoïdes sont désorbés dudit second solide poreux.

22. Un procédé selon la revendication 21, dans lequel lesdits premier et second solides poreux sont identiques, et/ou ladite absorption est effectuée sur lit fixe, un lit fluidisé fixe ou un lit en voie de déplacement, et/ou sont au moins présents deux lits de solides poreux, l'un fonctionnant en mode absorption et l'autre fonctionnant en mode désorption.

23. Un procédé d'extraction de composés diamantoïdes d'un fluide, comprenant les étapes:
(a) amener un fluide contenant une concentration récupérable de composés diamantoïdes;
(b) à soumettre le mélange gazeux à un procédé de séparation de façon à séparer au moins une partie desdits composés diamantoïdes dudit fluide,
dans lequel ledit fluide comprend une solution d'hydrocarbures substantielle desdits composés diamantoïdes, ladite solution contenant des fractions composés aromatiques, et dans lequel ledit procédé de séparation comprend la concentration des composés diamantoïdes dans lesdites solutions par extraction de la solution à l'aide de solvants sélectifs des composés aromatiques pour produire au moins un raffinat enrichi en diamantoïdes et un extrait enrichi en composés aromatiques, et à récupérer lesdits composés diamantoïdes dudit raffinat enrichi en composés diamantoïdes.

24. Un procédé selon la revendication 23, dans lequel le solvant sélectif à l'égard des dérivés composés aromatiques est au moins l'un de ceux choisis dans le groupe comprenant furfural, sulfolane, phénol, duosol et diméthyl formamide; et/ou
ladite extraction est effectuée à une température de l'ordre de 35 0 200°C (95 à 392°); et/ou
ladite extraction est effectuée à un taux d'extraction correspondant à un rapport de solution hydrocarbonée substantielle d'environ 2 sur 1 à 5 sur 1.

25. Un procédé selon la revendication 23 ou 24, dans lequel ladite solution formée substantiellement d'hydrocarbure contient en outre au moins un inhibiteur de corrosion.

26. Un procédé selon la revendication 25, dans lequel, dans lesdites partitions d'extraction se produit une séparation entre l'inhibiteur de corrosion avec lesdits composés aromatiques et les composés diamantoïdes s'en séparant.

27. Un procédé selon l'une quelconque des revendications 23 à 26, dans lequel ladite solution hydrocarbonée est formée par contact d'un dérivé minéral essentiellement hydrocarboné contenant des composés diamantoïdes avec un fioul de distillat riche en composés aromatiques de façon à faire passer les composés diamantoïdes du dérivé minéral dans ledit fioul.

28. Un procédé d'extraction d'un procédé diamantoïde d'un fluide comprenant les étapes consistant à:
(a) amener un fluide contenant une concentration récupérable de composés diamantoïdes;
(b) soumettre le fluide à un procédé de séparation de façon à séparer au moins une partie desdits composés diamantoïdes du courant gazeux,
dans lequel ledit procédé de séparation comprend les étapes de :
passage dudit courant enrichi en composés diamantoïdes entre deux surfaces espacées d'environ 0,0254 cm (0,01 inch) à une température supérieure à à celle du composé diamantoïde à point de fusion le plus bas contenu dans ledit courant de solvant enrichi en composés diamantoïdes jusqu'à pratiquement 260°C (500°F), et à un différentiel de température entre lesdites surfaces d'au moins environ 5,6°C (10°F) pendant une durée suffisante pour en récupérer un premier courant enrichi en composés diamantoïdes et un deuxième courant appauvri en composés diamantoïdes.

29. Un procédé selon la revendication 28, dans lequel lesdites surfaces sont maintenues à la température de l'ordre d'environ 10°C (50°F) à un ordre d'environ 260°C (500°F); et/ou
lesdites surfaces sont maintenues à un différentiel de température de l'ordre de 38°C (100°F) à environ 149°C (300°F).

30. Un procédé selon la revendication 28 ou 29, dans lequel le gaz naturel contenant une concentration substantielle de composés diamantoïdes est mise au contact d'un distillat aromatique pendant un temps suffisant pour extraire les composés diamantoïdes contenus dans ledit gaz naturel et pour former ledit fluide, lequel fluide comprend une solution de composés diamantoïdes, puis à faire passer ladite solution entre lesdites surfaces.

31. Un procédé selon l'une quelconque des revendications 28 à 30, dans lequel ledit fluide est passé de façon séquentielle entre une multiplicité des dites paires de surface en séries dans le sens du courant, et en ce qu'un produit enrichi en composés diamantoïdes est récupéré sur chaque paire de surface, ou en ce que ledit fluide est passé substantiellement simultanément entre une multiplicité desdites paires de surface, et en ce qu'on récupère un produit enrichi en composés diamantoïdes de chaque paire de surface.

32. Un procédé selon l'une quelconque des revendications 1 à 10, 17 à 22, ou 28 à 31, dans lequel le fluide est un courant gazeux, de préférence un courant de gaz naturel.

33. Un procédé selon l'une quelconque des revendications 1 à 22, ou 28 à 31, dans lequel le courant gazeux est obtenu à partir d'une source de gaz naturel, et en ce que le courant gazeux est fourni par retrait du gaz naturel contenant les composés diamantoïdes contenus dans ledit courant de gaz naturel produit.

34. Un procédé selon l'une quelconque des revendications 17 à 22 ou 28 à 31, dans lequel ledit fluide comprend du gaz naturel liquide, ou bien comprend une solution desdits composés diamantoïdes dans le fioul de distillat.
